(19) Europäisches Patentamt
European Patent Office
Office européen des brevets

(11) **EP 2 429 561 B1**

(12) **EUROPÄISCHE PATENTSCHRIFT**

(45) Veröffentlichungstag und Bekanntmachung des Hinweises auf die Patenterteilung:
**04.09.2013 Patentblatt 2013/36**

(21) Anmeldenummer: **10728587.6**

(22) Anmeldetag: **17.05.2010**

(51) Int Cl.:
*A61K 36/88* (2006.01)     *A61P 3/04* (2006.01)
*A61K 8/97* (2006.01)      *A61Q 19/00* (2006.01)
*A61Q 19/08* (2006.01)     *A61K 8/02* (2006.01)

(86) Internationale Anmeldenummer:
**PCT/DE2010/000555**

(87) Internationale Veröffentlichungsnummer:
**WO 2010/133211 (25.11.2010 Gazette 2010/47)**

(54) **ENTFÄRBTE EXTRAKTE DER PFLANZE PANDANUS CONOIDEUS**

DECOLORIZED EXTRACTS OF THE PLANT PANDANUS CONOIDEUS

EXTRAITS DÉCOLORÉS DE LA PLANTE PANDANUS CONOIDEUS

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **16.05.2009 DE 102009022046**

(43) Veröffentlichungstag der Anmeldung:
**21.03.2012 Patentblatt 2012/12**

(73) Patentinhaber: **Evoria GmbH**
**41460 Neuss (DE)**

(72) Erfinder: **SUSILO, Rudy**
**14089 Berlin (DE)**

(74) Vertreter: **Arth, Hans-Lothar**
**ABK Patent Attorneys**
**Jasminweg 9**
**14052 Berlin (DE)**

(56) Entgegenhaltungen:
**WO-A2-2007/048398**

- **OKID PARAMA ASTIRIN, MARTI HARINI, NOOR SOESANTI HANDAJANI: "The Effect of Crude Extract of Pandanus conoideus Lamb. var. Yellow Fruit on Apoptotic Expression of the Breast Cancer Cell Line (T47D)" BIODIVERSITAS, Bd. 10, Nr. 1, 1. Januar 2009 (2009-01-01) , Seiten 44-48, XP002607349 ISSN: 2085-4722**

**Beschreibung**

Die Erfindung betrifft entfärbte Extrakte aus der Frucht der Pflanze Pandanus conoideus, welche als kosmetisches sowie pharmazeutisches Mittel verwendet und beispielsweise zur Behandlung von immuno-dermatologischen Erkrankungen, Tumoren, Krebserkrankungen, rheumatoiden Erkrankungen, chronisch-rezidiven Entzündungen des Darms, degenerativen Erkrankungen, neurodegenerativen Erkrankungen, Inflammationen, gastrointestinalen Erkrankungen, Diabetes, kardiovaskulären Erkrankungen, immunologischen Erkrankungen, Infektionserkrankungen, ophthalmologischen und otologischen Erkrankungen eingesetzt werden können. Darüber hinaus beschreibt die vorliegende Anmeldung die Verwendung der entfärbten Extrakte als Sattmacher und Appetitzügler sowie allgemein als Diätmittel.

Pandanus conoideus ist eine endemische Pflanzenart aus Irian Jaya, Indonesien. Die Früchte dieser Pflanze finden Anwendung bei den Einheimischen als Nahrungsmittel, Nahrungsmittelergänzung, Viehfutter, natürlicher Farbstoff und als Heilmittel gegen verschiedenste Erkrankungen wie Würmererkrankungen und Blindheit.

Die traditionelle Zubereitung erfolgt durch langes Kochen der roten äußeren Fruchtschicht und anschließendem Zerreiben des Fruchtfleisches, um einen kernfreien Brei zu erhalten. Der daraus gewonnene ölige Brei wird als tägliches Lebensmittel oder als Heilmittel eingesetzt.

Die Pandanus conoideus ist eine kürbisartige längliche Frucht, welche im Inneren ein kernloses weißes helles Fruchtfleisch besitzt und darum herum befindet sich das rote Fruchtfleisch, worin sich auch die Kerne der Frucht befinden. Außen wird die zylindrische im Durchschnitt 1 m lange, 7,5 kg schwere und mit einem Durchmesser von 12 cm recht große Frucht von einer roten Schale umgeben.

Die aus dieser Frucht gewonnenen Extrakte und insbesondere die Extrakte aus dem roten äußeren Fruchtfleisch sind intensiv rot gefärbt. Diese rote Färbung schränkt die Verwendung der Extrakte und insbesondere solcher, die auch aus dem roten Fruchtfleisch gewonnen werden, bei der kosmetischen als auch der pharmakologischen, insbesondere topischen Verwendung stark ein.

Unter einer topischen Verwendung wird im Folgenden eine lokale therapeutische Anwendung verstanden. Besonders in der Dermatologie, also als äußere Anwendung auf zu behandelnder Haut, ist eine topische Verwendung von Bedeutung.

Für die äußere Anwendung vor allem im sichtbaren Bereich, d.h. auf Armen, Händen, Beinen, Füßen, im Gesicht und im Sommer auch auf dem ganzen Körper werden daher farblos Extrakte benötigt, welche ohne eine Rotfärbung der behandelten Haut eingesetzt werden können. Daher ist es besonders bevorzugt in einer dermatologischen oder kosmetischen Zusammensetzung einen entfärbten Pandanusextrakt einzusetzen, der soweit entfärbt ist, dass beim Einarbeiten der Pflanzenextrakte in kosmetische oder dermatologische Zusammensetzungen farblose Produkte entstehen, die keine sichtbare Färbung der Haut hinterlassen.

Erfindungsgemäß sind unter dem Begriff "Haut" bevorzugt die Haut selbst, insbesondere die menschliche Haut, daneben aber auch die Schleimhaut sowie Hautanhangsgebilde, sofern sie lebende Zellen umfassen, insbesondere Haarfollikel, das ventrale Epithel des Nagelbetts (Lectulus) sowie Talgdrüsen und Schweißdrüsen zu verstehen.

Natürlich sollen die nach Möglichkeit weitgehend entfärbten Extrakte ihre pharmakologische Aktivität beibehalten, d.h. der Entfärbungsprozess der Extrakte darf keinen Aktivitätsverlust der Extrakte bewirken und die pharmakologische Aktivität der entfärbten Extrakte sollte der pharmakologischen Aktivität der nicht entfärbten Extrakte weitgehend gleichkommen.

Somit stellt sich die Aufgabe der vorliegenden Erfindung darin, entfärbte Extrakte aus der Frucht der Pflanze Pandanus conoideus bereitzustellen, welche eine pharmakologische Aktivität aufweisen, die nahezu gleich der pharmakologischen Aktivität der unentfärbten Extrakte oder der Rohextrakte ist.

Diese Aufgabe wird durch die technische Lehre der unabhängigen Patentansprüche gelöst. Vorteilhafte Ausgestaltungen der Erfindung sind in den abhängigen Patentansprüchen, der Beschreibung sowie den Beispielen angegeben.

Überraschenderweise konnte festgestellt werden, dass die biologischen Aktivitäten der entfärbten Extrakte durch die Reduktion der Färbung wenig beeinflusst wurden. Dieses überraschende Ergebnis, d.h. eine nicht essentielle Verschlechterung der biologischen Aktivitäten insbesondere der anti-Krebswirkung und anti-Entzündungswirkung konnte auch noch bei starken Entfärbungen, d.h. bei einer Entfärbung von mindestens 65%, bevorzugt mindestens 70%, weiter bevorzugt mindestens 75%, noch weiter bevorzugt mindestens 80%, noch weiter bevorzugt mindestens 85% und am meisten bevorzugt mindestens 90% erzielt werden. Die Farbintensität der Extrakte wird photometrisch bei einer Wellenlänge von 478 nm bestimmt. Dazu wird ein Extrakt in Methanol gelöst, so dass eine Konzentration von 0,5 mg/ml vorliegt und seine Extinktion im Vergleich zum reinen Methanol photometrisch bestimmt. Ein erfindungsgemäßer, entfärbter Extrakt weist dabei bevorzugt eine Extinktion von maximal 0,28 weiter bevorzugt von maximal 0,22 weiter bevorzugt eine Extinktion von maximal 0,18, noch weiter bevorzugt eine Extinktion von 0,15 noch weiter bevorzugt eine Extinktion von 0,14, noch weiter bevorzugt eine Extinktion von 0,13, noch weiter bevorzugt eine Extinktion von 0,11, weiter bevorzugt eine Extinktion von 0,08 und am meisten bevorzugt eine Extinktion von maximal 0,075 auf.

Dabei ist eine Entfärbung nur vorteilhaft bei gefärbten Extrakten, d.h. Extrakten welche nur oder auch aus farbigen Bestandteilen der Frucht gewonnen wurden. Je nach Ausgangsmaterial kann auch direkt nach der Extraktion ein weitestgehend farbloser bzw. unterschiedlich stark gefärbter Extrakt vorliegen. Somit bezieht sich die Erfindung auf die Entfärbung von farbigen roten, rötlichen oder orangen Extrakten aus Pandanus conoideus.

Somit wird das erfindungsgemäße Verfahren auf gefärbte Extrakte angewendet, d.h. auf unentfärbte Extrakte oder Rohextrakte so wie sie nach der Extraktion der Frucht der Pflanze Pandanus conoideus ohne den Einsatz von Entfärbungsmitteln wie z.B. Oxidationsmitteln (oxidative Enzyme, Sauerstoff, Peroxide, Halogenverbindungen), Hydrierungsmitteln (Wasserstoff) oder UV-Strahlung erhalten werden. Die natürliche UV-Einstrahlung beispielsweise während der Trocknung der Pflanze oder der Pflanzenbestandteile unter dem Sonnenlicht wird nicht als Entfärbungsprozess angesehen, so dass eine durch natürliche UV-Einstrahlung hervorgerufene minimale Entfärbung der Pflanzenbestandteile unbeachtet bleibt.

Unter dem Begriff der "nicht essentiellen Verschlechterung der biologischen Aktivitäten" wird verstanden, dass die biologischen Aktivitäten und vor allem eine anti-Entzündungsaktivität und anti-Krebsaktivität als auch eine Aktivität gegen Hauterkrankungen (Dermatitis, Psoriasis, Ekzemen und der gleichen) der entfärbten oder teilweise entfärbten Extrakte weiterhin derart hoch oder derart gut ist, dass eine pharmakologische Anwendbarkeit der entfärbten oder teilweise entfärbten Extrakte weiterhin gegeben ist. Somit führt die Entfärbung nicht zu pharmakologisch unwirksamen oder pharmakologisch nicht mehr sinnvoll einsetzbaren Extrakten, sondern wider Erwarten zu Extrakten, welche im Wesentlichen in denselben Formulierungen, Konzentrationen, Dosierungen, Zeitintervallen und Wirkungen bei denselben Indikationen eingesetzt werden können wie die nicht entfärbten Extrakte bzw. die nicht nach dem erfindungsgemäßen Verfahren behandelte farbige Extrakte. Dieses Ergebnis war unerwartet, weil erfahrungsgemäß eine Entfärbung von farbigen pharmakologisch aktiven Extrakten immer mit einem deutlichen Aktivitätsverlust einhergeht, weil die unspezifischen Verfahren zur Entfärbung nicht nur die Farbstoffe sondern auch die Wirkstoffe zerstören, bzw. farbige Wirkstoffe mit der Farbe auch ihre Wirksamkeit verlieren. Da bisher angenommen wurde, dass die farbigen Stoffe in den Extrakten der Frucht der Pflanze Pandanus conoideus auch die pharmakologisch aktiven Substanzen sind, hätte bei einer Entfärbung oder teilweisen Entfärbung die pharmakologische Aktivität deutlich abnehmen müssen bis hin zu einem vollständigen Verlust der pharmakologischen Aktivität. Überraschend scheint die Frucht der Pflanze Pandanus conoideus jedoch oxidationsstabile, UV-stabile und Hydrierungsstabile Wirkstoffe zu enthalten, was für einen Fachmann überraschen ist.

Als geeignete Entfärbungsverfahren haben sich beispielsweise die folgenden erwiesen:

- Oxidation: Zufuhr von Sauerstoff über den Extrakt und/oder durch den Extrakt,
- Behandlung des Extraktes mit Peroxiden wie $Na_2O_2$, $CaO_2$, Diacylperoxide, Benzoylperoxid, $H_2O_2$, t-Butylhydroperoxid, Cumenhydroperoxid, Peroxycarbonsäure, meta-Chlorperbenzoesäure (mCPBA), Peressigsäure,
- Hydrierung mit Wasserstoff z.B. über $PtO_2$,
- Behandlung mit reduzierbaren Halogenverbindungen, halogenhaltige Bleichmittel wie Chlorwasser, Chlor, Brom, Iod, HClO (hypochlorige Säure oder unterchlorige Säure), Hypochloride, $ClO_2$, $HClO_2$, Chlorite, $HClO_3$, Chlorate, $HClO_4$, Perchlorate,
- Exposition des Extraktes gegenüber UV-Bestrahlung,
- Oxidation mittels oxidativer Enzyme.

Wasser sowie hohe Temperaturen beschleunigen den Entfärbungsprozess. Auch Aktivkohle kann zur weiteren Aufhellung der Extrakte sowohl vor als auch nach dem Entfärbungsprozess eingesetzt werden.

Mehrere Verfahren zur Entfärbung der nicht entfärbten Extrakte der Frucht der Pandanus conoideus wurden erfolgreich angewendet. Zu diesen Verfahren zählen beispielsweise die in Tabelle 1 genannten Methoden:

Tabelle 1: Methoden zur Farbreduktion des Pandanusextraktes (Details siehe Beispiele)

| Behandlung | Rest-Farbintensität in % (gemessen bei 478 nm) |
|---|---|
| Ein starker UVB-Strahler (30°C, 24 Stunden) | 8% |
| Benzoylperoxid (40 °C, 8 Stunden) | 16% |
| Extrakt in 70% Methanol-Wasser Lösung, . Sauerstoffzufuhr (40 °C, 48 Stunden) | 81 % |
| Hydrierung durch Zufuhr von Wasserstoff (40°C, 24 Stunden) | 73% |

Die Farbintensität der Extrakte kann am einfachsten durch eine starke UVB-Bestrahlung reduziert werden (Beispiel 6). Die Bestrahlung wird vorzugsweise ohne jegliche chemische Zusätze durchgeführt.

Die genaue Zusammensetzung der Farbstoffe in den Extrakten der Frucht der Pflanze Pandanus conoideus ist bisher unbekannt. Allgemein sind die meisten Wirkstoffe, die aus Pflanzen gewonnen werden jedoch Antioxidantien (Carlsen et al. 2010). Die bekanntesten sind Farbstoffe wie Carotinoide und Flavonoide. Daher ist es besonders überraschend, dass die gewonnenen Extrakte auch nach einer Entfärbung durch Oxidation bzw. Hydrierung oder UV-Bestrahlung noch derart wirksam sind. Daher muss angenommen werden, dass die vorteilhafte Wirkung der Extrakte auf einem anderen, bisher unbekannten Mechanismus beruht.

Aus der Frucht der Pflanze Pandanus conoideus lassen sich wie weiter unten beschrieben, verschiedene Extrakte gewinnen. Besonders die Extrakte aus dem roten Fruchtfleisch sind intensiv gefärbt und sind insbesondere als Rohextrakte geeignet, um bei den beschriebenen Entfärbungsverfahren eingesetzt zu werden.

Leichter gefärbt sind die Extrakte aus dem inneren hellen Fruchtfleisch und den Kernen, welche eine gelbliche Färbung aufweisen. Wird aus der gesamten Frucht ein Extrakt gewonnen, ohne das rote Fruchtfleisch von dem hellen inneren Fruchtfleisch zu trennen, so ist auch dieser Extrakt intensiv rot gefärbt und bedarf einer Entfärbung.

Die Extrakte aus der Frucht der Pflanze Pandanus conoideus haben wertvolle biologische Aktivitäten wie z.B. antientzündliche Aktivitäten, immunmodulatorische Wirkungen und die Wirkungen gegen humane Krebszellen.

Die starke Färbung der Extrakte verhindert und limitiert insbesondere die topische Anwendung, d.h. die Anwendung auf der Haut, vor allem dann, wenn höhere Konzentrationen an Extrakt benötigt werden oder eine Behandlung über einen längeren Zeitraum, vor allem bei chronischen Erkrankungen, durchgeführt wird. Überraschend war, dass die biologischen Aktivitäten der entfärbten Extrakte nach Anwendung der hierin beschriebenen Verfahren weitgehend erhalten blieb.

Somit richtet sich die Offenbarung auf entfärbte Extrakte aus der Frucht der Spezies Pandanus conoideus, wobei der entfärbte Extrakt eine Farbintensität von weniger als 50% im Vergleich zum unentfärbten Extrakt gemessen bei 478 nm aufweist. Des weiteren betrifft die Offenbarung entfärbte Extrakte, wobei der entfärbte Extrakt eine Farbintensität von weniger als 5% weiter bevorzugt von weniger als 15% im Vergleich zum unentfärbten Extrakt gemessen bei 478 nm aufweist. Der entfärbte Extrakt kann also eine Restfarbintensität von 0% bis 95%, bevorzugt von 0,1 % bis 85%, ebenfalls bevorzugt von 0,1 % bis 50% und noch weiter bevorzugt von 1 % bis 40% am meisten bevorzugt 10% bis 30% im Vergleich zum unentfärbten Extrakt gemessen bei 478 nm aufweisen.

Die entfärbten Extrakte besitzen mindestens 20%, bevorzugt mindestes 50%, weiter bevorzugt mindestes 70%, noch weiter bevorzugt mindestes 80% und und insbesondere bevorzugt mindestens 90% der antiinflammatorischen Wirkung des unentfärbten Extraktes. Die Bestimmung der antiinflammatorischen Wirkung wird in Beispiel 11 beschrieben.

Erfindungsgemäß besitzen die entfärbten Extrakte mindestens 20%, bevorzugt mindestes 50%, weiter bevorzugt mindestes 80% und insbesondere bevorzugt mindestes 90% der zytostatischen Wirkung des unentfärbten Extraktes. Die Bestimmung der zytostatischen Wirkung wird in Beispiel 10 beschrieben.

Die eingesetzten farbigen und zu entfärbenden Extrakte aus der Frucht der Pflanze Pandanus conoideus können durch bekannte Extraktionsverfahren gewonnen werden. Es gibt unterschiedliche Formen und Farben der Früchte von Pandanus conoideus, wie beispielsweise längliche rote Früchte, kurze rote Früchte, braune und gelbe Früchte. Aus allen diesen Früchten können entfärbbare Extrakte gewonnen werden.

Daher betrifft die vorliegende Erfindung auch Verfahren zur Herstellung eines entfärbten Extraktes umfassend die folgenden Schritte:

I) Bereitstellung eines farbigen Extraktes aus der Frucht der Spezies Pandanus conoideus,
II) Behandlung des farbigen Extraktes mittels

a) Bestrahlung durch UV-Licht, oder
b) Oxidation mit Peroxiden, oder
c) Oxidation mit Sauerstoff, oder
d) Oxidation mit reduzierbaren Halogenverbindungen, oder
e) Hydrierung mit Wasserstoff und Hydrierungskatalysator, oder
f) Biologischer Oxidation durch oxidative Enzyme

Es sei angemerkt, dass sich die Schritte a), b), c), d) e) und f) nicht einander ausschließen, sondern zwei oder mehr dieser Schritte auch in Kombination zeitgleich als auch nacheinander angewendet werden können. Zur oxidativen Entfärbung geeignete Enzyme (Schritt f)) können beispielsweise Oxidoreduktasen, Peroxidasen oder Dioxygenasen sein. Bevorzugt werden solche Enzyme, die aus Pilzen, Bakterien, Tieren und Pflanzen gewonnen werden. Ferner betrifft die vorliegende Erfindung entfärbte Extrakte, welche nach einem der hierin offenbarten Verfahren erhalten werden können.

Eine Möglichkeit der Gewinnung entfärbbarer Extrakte umfasst die folgenden Schritte:

1) durch die direkte Verpressung des roten Fruchtfleisches, woraus ein ölhaltiges oder öliges Produkt erhalten wird
2) durch eine Extraktion des roten Fruchtfleisches mit organischen Lösungsmitteln insbesondere lipophilen Lösungsmitteln, woraus ebenfalls ein ölhaltiges oder öliges Produkt erhalten wird

3) durch alkoholische Extraktion des roten Fruchtfleisches,

4) durch alkoholische Extraktion der Kerne,

5) durch die direkte Verpressung oder durch die Alkohol-Extraktion des roten Fruchtfleisches zusammen mit den Kernen

Ferner offenbart die vorliegende Anmeldung Verfahren zur Gewinnung von entfärbbaren Extrakten wie beispielsweise eines lipophilen Extraktes aus dem roten Fruchtfleisch der Früchte der Spezies Pandanus conoideus, der durch die direkte Verpressung, d.h. Kaltpressung des abgetrennten roten Fruchtfleisches bevorzugt ohne jegliche chemische Zusätze bei Temperaturen vorzugsweise unterhalb von 40°C oder durch Extraktion des abgetrennten zerkleinerten roten Fruchtfleisches mittels organischer Lösungsmittel bei Temperaturen vorzugsweise unterhalb von 40°C gewonnen wird. Die Extraktionen können grundsätzlich bei Temperaturen zwischen 4°C und 100°C durchgeführt werden. Insbesondere bevorzugt ist jedoch die Kaltpressung unterhalb von 40°C, da zum einen festgestellt worden ist, dass beim Kochen des roten Fruchtfleisches in Wasser oder einem organischen Lösungsmittel wichtige bioaktive Stoffe wie beispielsweise Tocopherole und andere Vitamine, Phytosterole etc. zerstört werden. Darüber hinaus wird ein durch Kochen hergestellter Extrakt sehr schnell ranzig. Durch die bevorzugte Kaltpressung erhält man überraschenderweise Extrakte, vorrangig Öle, welche mindestens ein Jahr und wahrscheinlich noch deutlich länger stabil sind. Die bisherigen Stabilitätsdaten zeigen eine Stabilität von zwei Jahren von Proben, welche bisher zwei Jahre lang bei 4 °C gelagert worden sind. Die Stabilitätsprüfung wird weiter fortgesetzt und es werden noch deutlich längere Stabilitäten erwartet.

Eine Temperaturerhöhung während des Extraktionsverfahrens oder des Verarbeitungsverfahrens des roten Fruchtfleisches über 40°C führt überraschenderweise zu einer Verringerung der Stabilität und Lagerstabilität des erhaltenen Extraktes. Je länger die Temperatur während der Extraktion des roten Fruchtfleisches über 40°C liegt und je höher die Temperatur während des Herstellungsprozesses des Öles aus dem rotem Fruchtfleisch über 40°C liegt, desto niedriger wird die Stabilität des Extraktes. Somit ist es wichtig, bei dem gesamten Herstellungsverfahren des Öls aus dem roten Fruchtfleisch darauf zu achten, dass die Extraktions- und Verarbeitungsbedingungen eine Temperatur von 60°C, bevorzugt 50°C und insbesondere bevorzugt 40°C nicht überschreiten und im Idealfall der gesamte Prozess der Extraktion und Ölgewinnung bei Raumtemperatur durchgeführt wird.

Für die Extraktion werden bevorzugt Hexan, Heptan, Cyclohexan, Chloroform, Methylenchlorid, Essigester, Diethylether, Petrolether, tert-Butylmethylether, THF, Methanol, Ethanol, Propanol, iso-Propanol, Butanol, iso-Butanol, sec-Butanol, Aceton und Mischungen dieser Lösungsmittel verwendet. Nach der Extraktion wird das Lösungsmittel vorzugsweise wieder entfernt und recyclisiert, um für weitere Extraktionen erneut verwendet zu werden. Das aufkonzentrierte Öl enthält die wirksamen Bestandteile des extrahierten Fruchtfleisches.

Ein weiteres bevorzugtes Extraktionsverfahren ist eine Alkohol und/oder AlkoholWasser- Extraktion des Fruchtfleisches samt der Kerne, indem zuerst das rote Fruchtfleisch samt der Kerne bei Temperaturen unterhalb von vorzugsweise 40°C zermahlen, mit Alkohol und /oder einer Alkohol-Wasser-Mischung unter Rühren vermischt und danach gepresst und filtriert wird. Vor dem Zermahlen können Fruchtfleisch und Kerne auch getrocknet werden. Die zerriebenen Keine samt verbliebenem zerriebenem Fruchtfleisch werden danach analog des Verfahrens zur Alkohol- und/oder Alkohol-Wasser-Extraktion des frischen Materials behandelt.

Ein weiteres Verfahren der vorliegenden Anmeldung betrifft die Herstellung eines hydrophilen Extraktes aus den abgetrennten Kernen der Frucht der Spezies Pandanus conoideus durch Zerkleinerung der Kerne bei Temperaturen unterhalb von vorzugsweise 40°C und Extraktion der zerkleinerten Kerne mit einem hydrophilen Lösungsmittel bei Temperaturen unterhalb von vorzugsweise 40°C. Auch hier gilt wie bei den anderen Extraktionen, dass die Extraktionen grundsätzlich bei Temperaturen zwischen 4°C und 100°C durchgeführt werden können, vorzugsweise jedoch Temperaturen von 60°C und insbesondere bevorzugt Temperaturen von 40°C nicht überschritten werden sollten.

Als hydrophiles Lösungsmittel werden vorzugsweise Wasser, Aceton, Tetrahydrofuran (THF), Methanol, Ethanol, Propanol, iso-Propanol, Butanol oder Mischungen dieser Lösungsmittel eingesetzt, wobei Wasser, Methanol und Ethanol oder Mischungen aus Wasser, Methanol und Ethanol bevorzugt sind. Nach eventuell mehrfacher Extraktion und Vereinigung der hydrophilen Extrakte, wird das Lösungsmittel wieder entfernt und erneut verwendet. Diese Schritte sollten vorzugsweise bei Temperaturen unterhalb von 40°C durchgeführt werden.

Ein weiteres Verfahren der vorliegenden Anmeldung betrifft die Herstellung eines hydrophilen Extraktes aus dem weißen Fruchtfleisch der Früchte der Spezies Pandanus conoideus durch eine direkte Verpressung des abgetrennten weißen Fruchtfleisches mit oder ohne Zusätze wie z.B. Zusatz von Antioxidantien bei Temperaturen vorzugsweise unterhalb von 40°C oder durch Trocknung und Zerkleinerung des abgetrennten weißen Fruchtfleisches bei Temperaturen unterhalb von vorzugsweise 40°C und anschließender Extraktion des getrockneten zerkleinerten weißen Fruchtfleisches mittels hydrophiler Lösungsmittel.

Als hydrophiles Lösungsmittel werden vorzugsweise Wasser, Aceton, Tetrahydrofuran (THF), Methanol, Ethanol, Propanol, iso-Propanol, Butanol oder Mischungen dieser Lösungsmittel eingesetzt, wobei Wasser, Methanol und Ethanol oder Mischungen aus Wasser, Methanol und Ethanol bevorzugt sind. Nach eventuell mehrfacher Extraktion des weißen bzw. hellen Fruchtfleisches und Vereinigung der hydrophilen Extrakte, wird das Lösungsmittel wieder entfernt und kann erneut verwendet werden. Alle Extraktionsschritte sollten vorzugsweise bei Temperaturen unterhalb von 40°C durchgeführt werden. Der aufkonzentrierte Extrakt enthält die pharmakologisch aktiven Bestandteile aus dem weißen Fruchtfleisch der Pandanus conoideus.

Die Frucht des Pandanus conoideus besteht aus einem inneren hellen Fruchtanteil und einem roten, kernhaltigen und ölhaltigen äußeren Fruchtanteil. Die vorliegende Anmeldung offenbart mögliche Extrakte, welche aus dieser Frucht gewonnen werden können. Diese Extrakte können sowohl allein als auch kombiniert angewendet werden, vorzugsweise als wässrige Lösung, als Öl oder als Feststoff. Für den Entfärbungsprozess werden jedoch vorzugsweise verdünnte Lösungen der farbigen und/oder entfärbbaren Extrakte eingesetzt. Bevorzugte Lösungs- oder Verdünnungsmittel sind Methanol, Ethanol, Aceton, Propanol, iso-Propanol, THF und Wasser.

Zur Gewinnung der bioaktiven entfärbbaren Extrakte werden die Früchte gereinigt und die rote, äußere Fruchtschicht von dem weißen, hellen, inneren Fruchtanteil abgetrennt. Die verschiedenen Extrakte können auf verschiedene Arten erhalten werden, wie beispielsweise in den Beispielen 1-5 offenbart ist.

Sämtliche Arbeitsschritte werden bevorzugt bei Temperaturen von 5 - 80°C, weiter bevorzugt bei 10°C bis 60°C, weiter bevorzugt bei 15 - 45°C, noch weiter bevorzugt bei 17 - 40°C und insbesondere bevorzugt bei 19°C - 38°C durchgeführt. Bevorzugt ist ferner, wenn die Verarbeitung bei Temperaturen unterhalb von 60°C, bevorzugt unterhalb von 50°C und insbesondere bevorzugt unterhalb von 40°C durchgeführt wird.

Während der Behandlung des farbigen Extraktes sollte die Temperatur unterhalb von 50°C bevorzugt unterhalb von 40°C liegen.

Die Herstellung der entfärbbaren Extrakte aus dem roten Fruchtfleisch oder aus den abgetrennten Kernen oder aus dem roten Fruchtfleisch samt den Kernen kann auch durch Extraktion mit überkritischen Fluiden (Kohlendioxid, Propan, Butan), vorzugsweise mit Kohlendioxid, mit oder ohne zusätzlichen Einsatz von Schleppmitteln (Methanol, Ethanol) durchgeführt werden. Mittels der vorgenannten Fluide lassen sich bevorzugt lipophile Extrakte erhalten, wobei durch gleichzeitige Verwendung von polaren Schleppmitteln auch weniger lipophile und eher polare bis hydrophile Extrakte gewonnen werden können.

Die durch Extraktion mit überkritischen Fluiden (Kohlendioxid, Propan, Butan) vorzugsweise mit Kohlendioxid gewonnenen Extrakte können natürlich einzeln oder in Kombination miteinander als auch in Kombination mit den nicht durch überkritische Fluide erhaltenen Extrakten eingesetzt werden. Zur Gewinnung der Extrakte werden vorzugsweise folgende Extraktionsverfahren angewendet: Ethanol-Wasser-Extraktion, Hexan-Extraktion und superkritische $CO_2$-Extraktion

Unter dem Begriff Extrakt oder entfärbbarer Extrakt soll sowohl die flüssige Phase nach einer einmaligen Extraktion als auch die kombinierten flüssigen Phasen von mehreren Extraktionen verstanden werden. Zudem soll der Begriff Extrakt auch die eingeengte flüssige Phase umfassen, bei welcher das Lösungsmittel teilweise, weitgehend oder vollständig entfernt worden ist, so dass Extrakt auch ein fester Rückstand bedeuten kann, der nach Entfernung des Lösungsmittels vorzugsweise unter vermindertem Druck und nach Trocknung der festen Bestandteile erhalten worden ist wie beispielsweise ein Lyophilisat. Ein nicht entfärbter Extrakt (100% Färbung) ist ein entfärbbarer Extrakt solange keine erfindungsgemäße Entfärbung stattgefunden hat, d.h. ein Extrakt der nach der entsprechenden Extraktion nicht weiter behandelt wurde. Ein entfärbarer bzw. nicht entfärbter Extrakt kann vor der erfindungsgemäßen Entfärbung gelagert werden.

Die erfindungsgemäßen, entfärbten Extrakte können in reiner Form oder aufbereitet als kosmetisches bzw. pharmakologisches Mittel zur Anwendung bei trockener Haut, Stoffwechselstörungen der Haut, Akne, geschädigter Haut, Alterungserscheinungen sowie zur Glättung der Haut und zur Steigerung der Feuchtigkeitsversorgung der Haut, sowie als Sattmacher, Appetitzügler und allgemein als Diätmittel eingesetzt werden.

Es wurde überraschend festgestellt, dass die tägliche orale Applikation des entfärbten oder teilweise entfärbten Pandanusexträkts die freiwillige Nahrungsaufnahme vermindert, wie in einem Tierversuch an Ratten nachgewiesen werden konnte (Beispiel 18).

Somit können die entfärbten oder teilweise entfärbten Extrakte in reiner Form oder aufbereitet als Diätmittel, alleine oder in Kombination als Schlankheitsmittel, Sattmacher, Appetitzügler oder Mittel zur Gewichtsbeibehaltung sowie Gewichtsreduktion eingesetzt werden. Unter den Begriff "Diätmittel" fallen sowohl Arzneimittel als auch Nahrungsergänzungsmittel.

Sattmacher sind Mittel, welche das Gefühl vermitteln, satt zu sein, ohne tatsächlich eine sättigende Menge an Nahrung zu sich genommen zu haben und Appetitzügler senken oder unterdrücken das Appetitgefühl, den sogenannten "Heißhunger", so dass man grundsätzlich weniger Lust hat, zu essen.

Derartige Mittel gibt es in großer Vielzahl auf dem Markt, wobei der Umfang an bekannten Nebenwirkungen ähnlich umfänglich ist. Die herkömmlichen Sattmacher oder Appetitzügler führen teilweise zu Kreislaufstörungen, erhöhtem Cholesterinspiegel, Bluthochdruck, Lungenhochdruck, Kopfschmerzen und Angstzuständen, um nur einige Nebenwirkungen zu nennen. Zudem wirken einige Appetitzügler stimmungsaufhellend und führen zu Abhängigkeiten, so dass beim Absetzen des Mittels rasch wieder eine Gewichtszunahme auftritt.

Derartige teils drastische Nebenwirkungen konnten bei den erfindungsgemäß eingesetzten, entfärbten oder teilweise entfärbten Extrakten der Pandanus conoideus nicht festgestellt werden. Die fehlenden schädlichen Nebenwirkungen sind somit genau so überraschend wie die gewichtsreduzierende Wirkung der Extrakte der vorliegenden Erfindung.

Somit betrifft die vorliegende Erfindung die Verwendung der erfindungsgemäßen Extrakte der Pandanus conoideus zur Herstellung eines Diätmittels zur Verwendung als Appetitzügler, Sattmacher, Schlankheitsmittel oder Mittel zur Gewichtsbeibehaltung oder Gewichtsreduktion. Die Mittel zur Gewichtsbeibehaltung werden insbesondere nach einer absolvierten Diät für einen längeren Zeitraum eingenommen, weil sich gezeigt hat, dass die Extrakte bei kontinuierlicher Einnahme in geringeren Konzentrationen einer erneuten Gewichtszunahme nach einer Diät entgegenwirken.

Der erfindungsgemäße Pandanusextrakt kann in der Therapie der Adipositas durch die Verminderung des Appetits eingesetzt werden. Die Wirkung tritt bereits 1 Tag nach der Applikation ein. Darüber hinaus eignen sich die entfärbten und teilweise entfärbten Pandanusextrakte als Appetitzügler und Sattmacher und zur Behandlung von Übergewicht, Fettleibigkeit oder Fresssucht und zur Verhinderung einer erneuten Gewichtszunahme nach absolvierter Diät.

Die Verträglichkeit der entfärbten oder teilweise entfärbten Extrakte ist sehr gut. Es wurden keinerlei Veränderungen der Tiere im Aussehen, Verhalten und im allgemeinen Zustand festgestellt. Die biochemischen Blutanalysen, sowie die histologischen Untersuchungen der Organe zeigten ebenfalls keinerlei Anomalien.

Durch die vorliegende Erfindung steht somit ein Mittel zur Körpergewichtsreduktion oder Verhinderung der Gewichtszunahme zur Verfügung. Die hierin beschriebenen Pandanusextrakte sind aufgrund ihrer hohen Sicherheit für die breite Anwendung als Massenprodukt für die Gewichtskontrolle gut geeignet.

Des Weiteren betrifft die vorliegende Erfindung kosmetische und dermatologische Zusammensetzungen zur Anwendung bei trockener Haut, Stoffwechselstörungen der Haut, Akne, geschädigter Haut, Alterungserscheinungen sowie zur Glättung der Haut und zur Steigerung der Feuchtigkeitsversorgung der Haut enthaltend einen erfindungsgemäßen entfärbten Extrakt.

Unter kosmetischer und/oder dermatologischer Zusammensetzung sind insbesondere Hautcremes, Hautlotionen, Emulsionen, Gele, Suspensionen, Salben, Öle sowie Balsame und sämtliche andere zur topischen Anwendung geeignete Formulierungen zu verstehen.

Weitere Anwendungsformen sind z.B. Sticks, Shampoos und Duschbäder. Der Formulierung können beliebige übliche Trägerstoffe, Hilfsstoffe und gegebenenfalls weitere Wirkstoffe zugesetzt werden. Vorzuziehende Hilfsstoffe stammen aus der Gruppe der Konservierungsstoffe, Antioxidantien, Stabilisatoren, Lösungsvermittler, Vitamine und Geruchsverbesserer.

Die erfindungsgemäße Zusammensetzung kann mindestens eine Substanz ausgewählt aus Vitaminen, Provitaminen oder Vitaminvorstufen der Vitamin B-Gruppe oder deren Derivaten enthalten. Panthenol, Pantolacton, Nicotinsäureamid sowie Biotin sind erfindungsgemäß ganz besonders bevorzugt.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe. Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten. Suspensionen können die üblichen Trägerstoffe wie flüssige Verdünnungsmittel, z.B. Wasser, Ethanol oderPropylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten. Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten. Weitere typisch kosmetische Anwendungsformen sind auch Lippenpflegestifte, Emulsions- und Wachs-Make up sowie Sonnenschutz-, Prä-Sun- und After-Sun-Präparate.

Die erfindungsgemäße kosmetische Zusammensetzung liegt in verschiedenen, für diese Anwendung üblicherweise verwendeten Darreichungsformen vor. So kann sie insbesondere als Lotion oder Emulsion, wie als Creme oder Milch (ON, WIO, ONIO, WION), in Form ölig-alkoholischer, ölig-wässriger oder wässrigalkoholischer Gele bzw. Lösungen, als feste Stifte vorliegen oder als Aerosol konfektioniert sein.

Unter Emulsionen versteht man heterogene Gemische, die aus zwei nicht miteinander mischbaren Flüssigkeiten bestehen, wobei eine der beiden Flüssigkeiten feinst verteilt als kleine Tröpfchen in der anderen Flüssigkeit vorliegt.

Die Ölphase der erfindungsgemäßen Zubereitung wird ausgewählt aus der Gruppe der Ester aus gesättigten und/oder ungesättigten, verzweigten und/oder unverzweigten Alkancarbonsäuren einer Kettenlänge von 3 bis 30 Kohlenstoff Atomen und gesättigten und/oder ungesättigten, verzweigten und/oder unverzweigten-Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Diese Esteröle werden vorteilhaft ausgewählt aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erycyloleat, Erucylerucat sowie synthetische, halbsynthetische und natürliche Gemische solcher Ester.

Weitere vorteilhafte Gruppen für die Ölphase umfassen verzweigte und unverzweigte Kohlenwasserstoffe und -wachse, Silikonöle, Dialkylether, gesättigte oder ungesättigte, verzweigte oder unverzweigte Alkohole sowie der Fettsäuretriglyceride, wie z.B. der Triglyceride gesättigter und/oder ungesättigter, verzweigter und/oder unverzweigter Alkancarbonsäuren einer Kettenlänge von 8 bis 24 C-Atomen. Vorteilhaft sind auch 2-Ethylhexylisostearatm Octyldodecanol, Isotridecylisononanoat, Isoeicosan, 2-Ethylhexylcocoat, $C_{12-15}$-Alkylbenzoat, Capryl-Caprinsäure-Triglycerid, sowie Dicaprylether. Jegliche Mischung aus Ölen und Wachsen kann erfindungsgemäß miteinander kombiniert werden.

Die erfindungsgemäße Zubereitung enthält vorteilhafterweise einen oder mehrere Emulgatoren, bevorzugt aus folgenden Gruppen: Sucroseester in Kombination mit Glycerylstearat und Glycerylstearatcitrat, Glycerylstearat in Kombination mit Ceteareth-20 und/oder Ceteareth-25, Ceteareth-6 in Kombination mit Stearylalkohol, Cetylstearylalkohol in Kombination mit PEG-40 Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl und/oder PEG-6 Caprylsäure/Caprinsäureglyceride, Glyceryloleat in Kombination mit Propylenglycol, PEG-9-Stearat, PEG-20 Stearat, PEG-30 Stearat, PEG-40 Stearat, PEG-100-Stearat, Ceteth-2. Ceteth-20, Polysorbate-20, Polysorbate-60, Polysorbate-65 und/oder Polysorbate-100, Glycerylstearat in Kombination mit PEG-100 Stearat, Glycerylmyristat, Glyceryllaurat, PEG-40-Sorbitanperoleat, Laureth-4, Ceteareth-3 und/oder Isostearylglycerylether, Cetylstearylalkohol in Kombination mit Natrium Cetylstearylsulfat, Laureth-23 und/oder Steareth-2, Glycerylstearat in Kombination mit PEG-30 Stearat, PEG-40 Stearat, Glycol Distearat, PEG-22-Dodecyl Glycol Copolymer, Polyglyceryl-2-PEG-4-Stearat, Ceteareth-12, Ceteareth-20, Ceteareth-30, Methyl-glucosesesquistearat, Steareth-10 und/oder PEG-20-Stearat, Steareth-2 in Kombination mit PEG-8 Distearat, Steareth-21, Steareth-20, Isosteareth-20, PEG-45/Dodecylgycol-Copolymer, Methoxy-PEG-22/Dodecylglycol-Copolymer, PEG-40-Sorbitanperoleat, PEG-40-Sorbitanperisostearat, PEG-20-Glycerylstearat, PEG-20-Glycerylstearat, PEG-8-Bienenwachs, Polyglyceryl-2-laurat, Isostearyldiglycerylsuccinat, Stearamidopropyl-PG-dimonium-chloridphosphat, Glycerylstearat SE, Ceteth-20, Triethylcitrat, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglyceryl-methylglucosedistearat, Kaliumcetylphosphat, Polyglyceryl-3 Methylglucose Distearate Isosteareth-10, Polyglyceryl-2-sesquiisostearat, Ceteth-10, Oleth-20 und/oder Cetylpalmitat, Cetylstearylalkohol in Kombination mit PEG-20 Stearat, PEG-30-Stearat, PEG-40-Stearat und/oder PEG-100-Stearat sowie Hydrogenated Lecithin.

Die erfindungsgemäßen Zusammensetzungen enthalten die Emulgatoren bevorzugt in Mengen von 0,1 bis 25 Gew.-%,insbesondere 0,5 - 15 Gew.-%, bezogen auf die gesamte Zusammensetzung.

Kosmetische und pharmazeutische Zusammensetzungen enthalten für den Fachmann bekannte Hilfs- und Zusatzstoffe, dazu gehören Konsistenzgeber, Füllstoffe, Parfum, Emulgatoren, Stabilisatoren, zusätzliche Wirkstoffe wie Vitamine, Lichtschutzmittel, Alkohol, Wasser, Salze sowie antimikrobielle oder proteolytische Substanzen,

Eine erfindungsgemäße Zusammensetzung kann Lipid-Partikel enthalten, in denen die Extrakt-Wirkstoffe transportiert werden. Die Formulierung der Zusammensetzung kann ferner kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und andere in der Kosmetik gewöhnlich

In einer besonders vorteilhaften Ausführungsform enthalten die erfindungsgemäßen, dermatologischen oder kosmetischen Zusammensetzungen mindestens ein filmbildendes, emulsionsstabilisierendes, verdickendes oder adhäsives Polymer, ausgewählt aus natürlichen und synthetischen Polymeren, die kationisch, anionisch, amphoter geladen oder nichtionisch sein können.

Erfindungsgemäß bevorzugt sind kationische, anionische sowie nichtionische Polymere. Unter den kationischen Polymeren bevorzugt sind Polysiloxane. Bevorzugte anionische Polymere, enthalten Carboxylat- und/oder Sulfonatgruppen und als Monomere zum Beispiel Acrylsäure, Methacrylsäure, Crotonsäure, Maleinsäureanhydrid und 2-Acrylamido-2-methylpropansulfonsäure. Geeignete nichtionische Polymere sind beispielsweise Polyvinylalkohole, die teilverseift sein können, z. B. die Handelsprodukte Mowiol@ sowie Vinylpyrrolidon/Vinylester-Copolymere und Polyvinylpyrrolidone, die z. B. unter dem Warenzeichen Luviskol@(BASF) vertrieben werden.

In einer weiteren bevorzugten Ausführungsform der Erfindung kann die Zusammensetzung durch Fettstoffe weiter optimiert werden. Geeignete Fettstoffe sind zum Beispiel:

- pflanzliche Öle, wie Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls,
- flüssige Paraffinöle, Isoparaffinöle und synthetische Kohlenwasserstoffe, z. B. 1,3-Di-(2-ethyl-hexyl)cyclohexan (Cetiola S) oder Polydecen,
- Fettsäuren, besonders lineare und/oder verzweigte, gesättigte und/oder ungesättigte Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen.

- Fettalkohole, besonders gesättigte, ein- oder mehrfach ungesättigte, verzweigte oder unverzweigte Fettalkohole
- Hydroxycarbonsäurealkylester, wobei die Vollester der Glycolsäure, Milchsäure, Äpfelsäure, Weinsäure oder Citronensäure bevorzugt sind, aber auch Ester der ß-Hydroxypropionsäure, der Tartronsäure, der D-Gluconsäure,
- Wachse, insbesondere Insektenwachse wie Bienenwachs und Hummelwachs, Pflanzenwachse wie Candelillawachs und Carnaubawachs, Fruchtwachse, Ozokerit, Mikrowachs, Ceresin und Paraffin

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer einem entfärbten Extrakt aus Pandanus conoideus beispielsweise Fettalkohole, Fettsäuren, Fettsäureester, insbesondere Triglyceride von Fettsäuren, Lanolin (Wollwachs), natürliche und synthetische Öle oder Wachse und Emulgatoren in Anwesenheit von Wasser enthält.

Die erfindungsgemäße kosmetische Zusammensetzung kann auch als alkoholisches Gel vorliegen, welches einen oder mehrere Niedrigalkohole oder - polyole, wie Ethanol, Propylenglykol oder Glycerin, und ein Verdickungsmittel, wie Kieselerde umfasst. Die ölig-alkoholischen Gele enthalten außerdem natürliches oder synthetisches Öl oder Wachs. Neben einem entfärbten Pandanus-Extrakt und den üblicherweise verwendeten Lösungsmitteln können solche Gele weiterhin organische Verdickungsmittel enthalten, wie z.B. Gummiarabikum, Xanthangummi, Natriumalginat, Cellulose-Derivate, bevorzugt Methylcellulose, Hydroxymethylcellulose, Hydroxyethylcellulose, Hydroxypropylcellulose, Hydroxypropylmethylcellulose oder anorganische Verdickungsmittel, wie z.B. Aluminiumsilikate, beispielsweise Bentonit oder ein Gemisch aus Polyethylenglykol und Polyethylenglykolstearat oder -distearat.

Eine erfindungsgemäße kosmetische Zusammensetzung kann folgende Konservierungsmittel enthalten: Phenoxyethanol, Formaldehydlösung, Parabene, Pentadiol oder Sorbinsäure.

Ferner können die erfindungsgemäßen, entfärbten Extrakte in reiner Form oder aufbereitet als pharmazeutisches Mittel zur Behandlung und Prophylaxe von immuno-dermatologischen Erkrankungen, Tumoren, Krebserkrankungen, rheumatoiden Erkrankungen, chronisch-rezidiven Entzündungen des Darms, degenerativen Erkrankungen, neurodegenerativen Erkrankungen, Inflammationen, gastrointestinalen Erkrankungen, Diabetes, kardiovaskulären Erkrankungen, immunologischen Erkrankungen, Infektionserkrankungen, ophthalmologischen und otologischen Erkrankungen verwendet werden.

Bei der Anwendung der erfindungsgemäß entfärbten Extrakte der Spezies Pandanus conoideus konnten keinerlei unerwünschte Nebenwirkungen festgestellt werden, so dass die Extrakte als sehr gut verträglich eingestuft werden und für eine Langzeitanwendung wie z.B. bei Neurodermitis erforderlich, geeignet sind.

Neurodermitis wird auch als atopisches Ekzem, endogenes Ekzem, chronisch konstitutionelles Ekzem, Asthmaekzem, Prurigo Besnier oder atopische Dermatitis bezeichnet. Die Krankheit ist chronisch und nicht ansteckend. Ferner gilt sie als behandelbar, jedoch zur Zeit noch immer als unheilbar. Die sich in roter, trockener, schuppender und manchmal durch nässende Ekzeme auf der Haut äußernde Krankheit wird in der Regel durch die topische Anwendung entzündungshemmender Stoffe behandelt.

Dabei ist vor allem bei äußerlicher Anwendung der Extrakte, eine weitestgehende Entfärbung vorteilhaft, da eine unschöne Rotfärbung der Haut vermieden werden kann. Dies gilt insbesondere zur Behandlung von chronischen Erkrankungen vor allem von exponierten Körperbereichen wie dem Gesicht.

Des Weiteren betrifft die vorliegende Erfindung die Verwendung der entfärbten Extrakte der Pandanus conoideus zur Herstellung eines pharmazeutischen Mittels zur Behandlung und Prophylaxe von immuno-dermatologischen Erkrankungen, Tumoren, Krebserkrankungen, rheumatoiden Erkrankungen, chronisch-rezidiven Entzündungen des Dickdarms, degenerativen Erkrankungen, neurodegenerativen Erkrankungen, Inflammationen, gastrointestinalen Erkrankungen, Diabetes, kardiovaskulären Erkrankungen, immunologischen Erkrankungen, Infektionserkrankungen, ophthalmologischen und otologischen Erkrankungen.

Ein erfindungsgemäß entfärbter Extrakt oder eine Kombination aus mehreren erfindungsgemäß entfärbten Extrakten aus der Pandanus conoideus wird/werden vorzugsweise zur Herstellung einer pharmazeutischen, dermatologischen oder kosmetischen Zusammensetzung verwendet, welche den entfärbten Extrakt oder die entfärbten Extrakte aus Pandanus conoideus zusammen mit mindestens einem pharmakologisch oder kosmetisch verträglichen Hilfsstoff, Trägerstoff und/oder Lösungsmittel enthält.

Die erfindungsgemäßen entfärbten Extrakte können alleine oder in Kombination mit weiteren Wirkstoffen, Vitaminen, Spurenelementen, Mineralien so wie sie entfärbt wurden oder in Form pharmazeutischer Formulierungen und Zusammensetzungen gegen diverse, hierin beschriebene Erkrankungen eingesetzt werden.

Die pharmazeutische Zusammensetzung enthaltend einen entfärbten Extrakt oder mehrere entfärbte Extrakte aus Pandanus conoideus kann mit den üblichen festen oder flüssigen Trägerstoffen oder Verdünnungsmitteln und den üblicherweise verwendeten pharmazeutischen Hilfsstoffen entsprechend der gewünschten Applikationsart mit einer geeigneten Dosierung in bekannter Weise hergestellt werden. Solche Darreichungsformen sind beispielsweise Tabletten, Minitabletten, Mikrotabletten, Filmtabletten, Schichttabletten, Dragees, Kapseln, Mikrokapseln, Mikropellets und Pellets, Pillen, Granulate, Pulver, Puder, Lösungen Tropfen, Säfte, Suspensionen, Suppositorien, Klysma, Emulsionen, Dispersionen, Creme, Gele, Salben, Sirup oder Depotformen oder Inhalationslösungen bzw. Inhalationspulver. Zudem umfassen die erfindungsgemäßen pharmazeutischen Zusammensetzungen Formulierungen wie Schichttabletten zur kontrollierten und/oder kontinuierlichen Freisetzung des oder der Wirkstoffe sowie Mikroverkapselungen als spezielle Darreichungsformen.

Derartige Zubereitungen sind unter anderem für die Inhalation oder die intravenöse, intraperitoneale, intramuskuläre, subkutane, orale, rektale, transdermale, topikale, intradermale, intragastrale, intrakutane, intravaginale, intranasale, intrabuccale, perkutane oder sublinguale Verabreichung geeignet.

Besonders vorteilhafte Darreichungsformen sind die orale, rektale und topische Applikation, die Injektion, die Infusionen als auch die Inhalation.

Entsprechende Tabletten können beispielsweise durch Mischen der/des erfindungsgemäß einsetzbaren Extrakte(s) mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Hydroxypropylmethylcellulose, Celluloseacetatphthalat oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talkum, Titandioxid oder Zucker hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäß hergestellten Extrakt können zusätzlich geschmacksverbessernde Mittel wie Saccharin, Cyclamat oder Zucker sowie Aromastoffe wie Vanillin oder Orangenextrakt enthalten. Sie können außerdem Suspendierhilfsstoffe wie Natriumcarboxymethylcellulose oder Konservierungsmittel wie p-Hydroxybenzoate enthalten. Den Extrakt enthaltende Kapseln können beispielsweise hergestellt werden, indem man den Wirkstoff mit einem inerten Träger wie Milchzucker oder Sorbit mischt und in Gelatinekapseln einkapselt.

Der Extrakt kann auch direkt in Weichgelatine-Kapseln verarbeitet werden.

Geeignete Suppositorien lassen sich beispielsweise durch Vermischen mit dafür vorgesehenen Trägermitteln wie Neutralfetten oder Polyethylenglykol bzw. Derivaten davon herstellen.

Der Extrakt kann auch als Klistier (rektale Instillation) verabreicht werden.

Bestimmte Formulierungen sind unter anderem für die Inhalation oder die intravenöse, intraperitoneale, intramuskuläre, subkutane, orale, rektale, transdermale, topikale, intradermale, intragastrale, intrakutane, intravaginale, intranasale, intrabuccale, perkutane oder sublinguale Verabreichung geeignet. Als pharmakologisch verträgliche Träger können beispielsweise Lactose, Stärke, Sorbitol, Sucrose, Cellulose, Magnesiumstearat, Dicalciumphosphate, Calciumsulfate, Talk, Mannitol, Ethylalkohol und dergleichen eingesetzt werden. Puder als auch Tabletten können zu 5 bis 95% aus einem derartigen Träger bestehen.

Als Bindemittel können zudem Stärke, Gelatine, natürliche Zucker, natürliche als auch synthetische Gummis wie beispielsweise Akaziengummi oder Guar-Gummi, Natriumalginat, Carboxymethyl-Cellulose, Polyethylenglycol und Wachse eingesetzt werden. Als Gleitmittel können Borsäure, Natriumbenzoat, Natriumacetat, Natriumchlorid, und dergleichen dienen.

Ferner können den pharmazeutischen Zusammensetzungen noch Sprengmittel, Farbstoffe, Geschmacksstoffe und/oder Bindemittel zugesetzt werden.

Flüssige Formulierungen umfassen Lösungen, Suspensionen, Sprays und Emulsionen. Beispielsweise Injektionslösungen auf Wasserbasis oder Wasser-Propylenglycol-Basis für parenterale Injektionen.

Für die Zubereitung von Suppositorien werden bevorzugt niedrigschmelzende Wachse, Fettsäureester und Glyceride eingesetzt.

Kapseln werden beispielsweise aus Methylcellulose, Polyvinylalkohol oder denaturierter Gelatine oder Stärke hergestellt.

Als Sprengmittel können Stärke, Natrium-Carboxymethylstärke, natürliche und synthetische Gummis wie beispielsweise Johannisbrotkernmehl, Karaya, Guar, Tragant und Agar sowie Cellulosederivate wie Methylcellulose, Natrium-Carboxymethylcellulose, mikrokristalline Cellulose sowie Alginate, Tonerden und Bentonite verwendet werden. Diese Bestandteile können in Mengen von 2 bis 30 Gew.-% eingesetzt werden.

Als Bindemittel können Zucker, Stärke aus Korn, Reis oder Kartoffeln, natürliche Gummis wie Akaziengummi, Gelatine, Tragant, Alginsäure, Natriumalginat, Ammonium-Calcium-Alginat, Methylcellulose, Natrium-Carboxymethylcellulose, Hydroxypropyl-methylcellulose, Polyvinylpyrrolidon sowie anorganische Verbindungen wie Magnesium-Aluminum-Silicate zugesetzt werden. Die Bindemittel könne in Mengen von 1 bis 30 Gew.-% zugesetzt werden.

Als Gleitmittel können Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natriumoleat, Polyethylenglycol und Aminosäuren wie Leucin eingesetzt werden. Derartige Gleitmittel können in Mengen von 0,05 bis 15 Gew.-% verwendet werden.

Die erfindungsgemäße pharmazeutische Formulierung wird insbesondere in der Krebstherapie, bei rheumatoiden Erkrankungen sowie bei chronisch-rezidivierenden Entzündungen des Dickdarms eingesetzt. Bevorzugt ist zudem, die erfindungsgemäße pharmazeutische Formulierung in Kombination mit einem Antikrebsmittel zu verabreichen oder mit einer herkömmlichen Chemotherapie, mit Antirheumatika oder mit Medikamenten, die bei entzündlichen Dickdarmerkrankungen eingesetzt werden, zu kombinieren. Ferner ist bevorzugt, der erfindungsgemäßen pharmazeutischen Formulierung ein Vitamin und/oder mindestens einen antiproliferativen, antiangiogenen, antiinflammatorischen, antiphlogistischen, cytostatischen und/oder cytotoxischen Wirkstoff zuzusetzen.

Als Vitamine können Vitamin A, Vitamin C (Ascorbinsäure), Vitamin D, Vitamin H, Vitamin K, Vitamin E, VitaminB1, VitaminB2, VitaminB3, VitaminB5, VitaminB6, VitaminB12, Thiamin, Riboflavin, Niacin, Pyridoxin und Folsäure verwendet werden.

Als antiproliferative, antiangiogene, cytostatische und/oder cytotoxische Wirkstoffe können der pharmazeutischen Zusammensetzung Chlorethamin, Cyclophosphamid, Trofosfamide, Ifosfamid, Melphalan, Chlorambucil, Busulfan, Thiotepa, Carmustin, Lomustin, Dacarbazin, Procarbazin, Temozolomid, Treosulfan, Estramustin, Nimustin, Daunorubicin, Doxorubicin, Adriamycin, Dactinomycin, Mitomycin C, Bleomycin, Epirubicin (4-Epi-Adriamycin), Idarubicin, Dactinomycin, Mitoxantron, Mitomycin-C, Plicamycin, Amsacrin, Actinomycin D, Methotrexat, 5-Fluorouracil, 6-Thioguanin, 6-Mercaptopurin, Fludarabin, Cladribin, Pentostatin, Gemcitabin, Cytarabin, Azathioprin, Raltitrexed, Capecitabin, Cytosinarabinosid, Tioguanin, Mercaptopurin, Vincristin, Vinblastin, Vindesin, Etoposid, Teniposid, Cisplatin, Carboplatin, Oxaliplatin, Vinca-Alkaloide, Venorelbin, Etoposid, Teniposid, Camptothecin, Irinotecan, Paclitaxel, Docetaxel, Hydroxycarbamide (Hydroxyurea), Imatinib, Miltefosin, Amsacrin, Topotecan

(Topoisomerase-I-Inhibitor), Bexaroten, Tretinoin, Asparaginase, Trastuzumab, Alemtuzumab, Rituximab, Glucocorticoide (Prednison Prednisolon, Dexamethason), Oestrogene (Fosfestrol, Estramustin), LHRH (Buserelin, Goserelin, Leuprorelin, Triptorelin), Flutamid, Cyproteronacetat, Tamoxifen, Toremifen, Aminoglutethimid, Formestan, Exemestan, Letrozol, Anastrozol, Interleukin-2, Interferon-$\alpha$, Erythropoietin, G-CSF, Rituximab, Efitinib, Ibritumomab, Levamisol, Indomethacin, Diclofenac, Ketoprofen, Ibuprofen, Flurbiprofen, Nabumeton, Cox-2-Hemmer, Mesalazin, Sulfasalazin, Olsalazin, Infliximab und/oder Retinoide zugesetzt werden.

Als antiinflammatorische und antiphlogistische Substanzen können der pharmazeutischen Zusammensetzung nicht steroidale Antirheumatika wie Acetylsalicylsäure, Essigsäurederivate wie Indomethacin, Diclofenac oder Propionsäurederivate wie Ibuprofen, Flurbiprofen, Ketoprofen, Naproxen, Tiaprofensäure oder sonstige Verbindungen wie Nabumeton und Cox-2-Inhibitoren zugesetzt werden. Weitere Beispiele für antiinflammatorische Wirkstoffe sind Alcofenac, Aceclofenac, Sulindac, Tolmetin, Etodolac, Fenopren, Thiaprofensäure, Meclofenamsäure, Meloxicam, Tenoxicam, Lornoxicam, Nabumeton, Acetaminophen, Phenacetin, Ethenzamid, Sulpyrin, Mefanamsäure, Flufenamsäure, Diclofenacnatrium, Loxoprofennatrium, Phenylbutazon, Indomethacin, Oxaprozin, Flurbiprofen, Fenbufen, Pranoprofen, Floctafenin, Piroxicam, Epirizol, Tiaramidhydrochlorid, Zaltoprofen, Gabexatmesylat, Camostatmesylat, Ulinastatin, Colchicin, Probenecid, Sulfinpyrazon, Benzbromaron, Allopurinol, Salicyläure, Atropin, Scopolamin, Levorphanol, Ketorolac, Tebufelon, Tenidap, Clofezon, Oxyphenbutazon, Prexazon, Apazon, Benzydamin, Bucolom, Cinchopen, Clonixin, Ditrazol, Epirizol, Fenoprofen, Floctafeninl, Glaphenin, Indoprofen, Nifluminsäure, Suprofen, Bufexamac, Dexamethason, Hexestrol, Methimazol, Betamethason, Triamcinolon, Fluocinonid, Prednisolon, Methylprednisolon, Hydrocortison, Fluorometholon, Beclomethasondipropionat, Estriol, Clobetasol, Diflorasondiacetat, Halbetosalpropionat, Amicinonid, Desoximetason, Halcinonid, Mometasonfuroat, Fluticasonpropionat, Flurandrenolid, Clocortalon, Predincarbat, Aclometasondipropionat und Desonid.

Auch Substanzen gegen chronisch-rezidivierende Entzündung des Darms (Colitis ulcerosa und Morbus Crohn) wie Mesalazin, Sulfasalazin, Olsalazin, Glucocorticoide (Prednisolon, Prednison, Dexamethason), Ciclosporin, Infliximab können der pharmazeutischen Zusammensetzung zugesetzt werden.

Die erfindungsgemäßen pharmazeutischen Formulierungen ohne oder auch mit mindestens einem antiproliferativen, antiangiogenen, antiinflammatorischen, antiphlogistischen, cytostatischen und/oder cytotoxischen Wirkstoff oder die entfärbten Extrakte werden vorzugsweise zur Prophylaxe und Behandlung von rheumatoiden Erkrankungen, chronisch-rezidivierenden Entzündungen des Dickdarms, Tumoren und Krebs und insbesondere von akuter und chronischer myeloischer Leukämie, akuter und chronischer lymphatischer Leukämie, Adenokarzinomen, Aderhautmelanom, Akustikusneurinom, Ampullenkarzinom, Analkarzinom, Astrozytomen, Basaliom, Bauchspeicheldrüsenkrebs, Bindegewebstumor, Blasenkrebs, Bronchialkarzinom, nicht-kleinzelligem und kleinzelligem Bronchialkarzinom, Brustkrebs, Burkitt-Lymphom, Corpuskarzinom, CUP-Syndrom, Dickdarmkrebs, Dünndarmkrebs, Dünndarmtumoren, Eierstockkrebs, Endometriumkarzinom, Ependymom, Epithel-Krebsarten, Ewing-Tumoren, gastrointestinalen Tumoren, Gallenblasenkrebs, Gallenkarzinomen, Gebärmutterkrebs, Gebärmutterhalskrebs, Glioblastomen, gynäkologischen Tumoren, Hals-, Nasen- und Ohrentumoren, hämatologischen Neoplasien, Haarzell-Leukämie, Harnröhrenkrebs, Hautkrebs, Hirntumoren (Gliome), Hirnmetastasen, Hodenkrebs, Hypophysentumor, Karzinoiden, Kaposi-Sarkom, Kehlkopfkrebs, Keimzellentumor, Knochenkrebs, kolorektalem Karzinom, Kopf-Hals-Tumoren (Tumore des Hals- Nasen- und Ohrenbereichs), Kolonkarzinom, Kraniopharyngeomen, Krebs im Mundbereich und auf der Lippe, Leberkrebs, Lebermetastasen, Leukämie, Lidtumor, Lungenkrebs, Lymphdrüsenkrebs (Hodgkin/Non-Hodgkin), Lymphomen, Magenkrebs, malignem Melanom, malignem Neoplasma, Malignome des Magen-Darm-Traktes, Mammakarzinom, Mastdarmkrebs, Medulloblastomen, Melanom, Meningeomen, Morbus Hodgkin, , Nasenkrebs, Neurinom, Neuroblastom, Nierenkrebs, Nierenzellkarzinomen, Non-Hodgkin-Lymphomen, Oligodendrogliom, Ösophaguskarzinom, osteolytischen Karzinomen. u. osteoplastischen Karzinomen, Osteosarkom, Ovarial-Karzinom, Pankreaskarzinom, Peniskrebs, Plasmozytom, Plattenepithelkarzinomen des Kopfes und Halses, Prostatakrebs, Rachenkrebs, Rektumkarzinom, Retinoblastom, Scheidenkrebs, Schilddrüsenkarzinom, Schneeberger Krankheit, Speiseröhrenkrebs, Spinaliom, T-Zell-Lymphom (Mycosis fungoides), Thymom, Tubenkarzinom, Tumoren des Auges, Urethrakrebs, urologischen Tumoren, Urothelkarzinom, Vulvakrebs, Warzenbeteiligung, Weichteiltumoren, Weichteilsarkom, Wilms Tumor, Zervixkarzinom und Zungenkrebs eingesetzt.

Des Weiteren können die erfindungsgemäßen Extrakte, als auch die erfindungsgemäßen pharmazeutischen Formulierungen zur Prophylaxe und/oder Behandlung von rheumatoiden Erkrankungen, degenerativen Erkrankungen, neurodegenerativen Erkrankungen, Inflammation (Entzündungen), chronischen Entzündungen des Darms, Diabetes, kardiovaskulären Erkrankungen, Autoimmun-Erkrankungen und Infektionserkrankungen benutzt werden.

Als Beispiele für rheumatoide Erkrankungen, degenerative Erkrankungen, neurodegenerative Erkrankungen, Inflammation (Entzündungen), Diabetes, kardiovaskuläre Erkrankungen, Autoimmun-Erkrankungen und Infektionserkrankungen können genannt werden: rheumatoide Arthritis, Osteoarthritis, chronische Lungenentzündung, Asthma, Lupus (SLE), Atherosclerosis, Nephritis, Psoriasis, Arthritische Psoriasis, Rheumatoide Arthritis, Osteoarthritis, Allergien, Crohn'sche Krankheit, Ischemia, Bowel Krankheit, Tuberkulose, interstitiale Cystitis, Sonnenbrand; Alzheimer, Parkinson Krankheit, Huntington Krankheit, amyotrophische laterale Sclerose, Retinitis pigmentosa, Shy Drager Syndrom; Autoimmunerkrankungen, Osteoporose, ulcerative Colitis, Ekzeme, multiple Sclerosis (MS); Diabetes Mellitus Typ 1, Diabetes Mellitus Typ 2; HIV, AIDS, Varicella-Zoster-Virus-Infektionen, Creutzfeldt-Jakob Krankheit (CJD), Konjunktivitis, Encephalitis, Epstein-Barr-Virus-Infektionen, Dermatitis, Helicobacter pylori Infektionen, Herpesvirus-Infektionen, Influenza, Meningitis, Ulcer; Herzinfarkt, Angina Pectoris, Arteriosklerose, kongenitale Herzfehler, Hämatome, pulmonare Hypertension, Myocarditis, Pericarditis, Phlebitis, Restenose; Stenose, Herzinsuffizienz und Thrombose.

Bevorzugt werden die erfindungsgemäßen entfärbten oder teilweise entfärbten Extrakte bei Dermatitis, Arthritischer Psoriasis, Rheumatoider Arthritis und Osteoarthritis eingesetzt.

Die hierin beschriebenen Extrakte können alleine oder in Kombination mit anderen erfindungsgemäßen Extrakten und/oder in Kombination mit weiteren Wirkstoffen, Vitaminen, Spurenelementen, Mineralien so wie sie gewonnen wurden oder in Form diätetischer Formulierungen und Zusammensetzungen gegen Übergewicht, Fettleibigkeit, Gewichtszunahme bei Über- oder Normalgewichtigen sowie gegen Fresssucht und fehlendes Sättigungsgefühl eingesetzt werden.

Die Diätmittel und diätetischen Formulierungen werden als Schlankheitsmittel, Appetitzügler und Sattmacher bei Diäten sowie zur Behandlung von Fettleibigkeit, Übergewicht und Fresssucht eingesetzt und können aufgrund ihrer guten Verträglichkeit und der fehlenden Nebenwirkungen und der fehlenden Suchtgefahr auch prophylaktisch eingesetzt werden. Derartige prophylaktische Verwendungen der Extrakte und Diätmittel umfassen insbesondere eine kontinuierliche Einnahme der Extrakte oder Diätmittel nach einer erfolgreich durchgeführten oder mit Gewichtsverlust absolvierten Diät, um eine erneute Gewichtszunahme zu verhindern und den Jojo-Effekt zu vermeiden. Als Jojo-Effekt-wird die Beobachtung bezeichnet, dass nach einer Diät mit Gewichtsverlust bei Beendigung der Diät eine stärkere Gewichtszunahme beobachtet wird, welche wiederum eine ausgedehntere Diät nach sich zieht und man das nach einer Diät erreichte Gewicht nicht halten kann.

Die Dosierung der Extrakte kann auf Grund der nicht nachweisbaren Nebenwirkungen oder Toxizität in einem breiten Bereich gewählt werden.

Im Tierversuch mit Ratten lag die Dosierung bei 10 mg - 5.000 mg /kg Körpergewicht (KG), vorzugsweise bei 50-2000 mg/kg KG und insbesondere bevorzugt bei 100-1.000 mg/kg KG.

Beim Menschen liegt die übliche Dosierung bei 1 mg - 200 mg /kg Körpergewicht (KG), vorzugsweise bei 2-150 mg/kg KG und insbesondere bevorzugt bei 4-70 mg/kg KG. Pro Applikation wird vorzugsweise eine Menge von 10 mg - 20 000 mg Extrakt oder ein Diätmittel enthaltend 10 mg - 20 000 mg Extrakt und weiter bevorzugt eine Menge von 100 mg - 15.000 mg Extrakt oder ein Diätmittel enthaltend 100 mg - 15.000 mg Extrakt und insbesondere bevorzugt ein Diätmittel enthaltend 500 mg - 5.000 mg Extrakt verabreicht. Üblicherweise werden 1 bis 10, vorzugsweise 3 bis 6 Dosen pro Tag verabreicht.

Tabelle 6 zeigt, dass das Körpergewicht der Tiere in der Kontrollgruppe durchschnittlich um 5 g pro Tag zunimmt. Innerhalb von 5 Tagen nimmt das Körpergewicht um 16% zu.

In der mit 250 mg/kg KG Pandanusextrakt behandelten Gruppe steigt das Körpergewicht im Durchschnitt täglich um 0,6 g. Nach 7-tägiger Behandlung steigt das Körpergewicht um 2,2%.

Es konnte keine signifikante Veränderungen der relativen Lebergewichte (des Verhältnisses von Leber- zu Körpergewicht) gemessen werden und die Leberenzym-Parameter (SGOT, SGPT) sowohl in der Kontrollgruppe als auch in der behandelten Gruppe waren unverändert. Dies deutet auf die gute Verträglichkeit und die Sicherheit der Anwendung des Extraktes hin.

Die Ergebnisse zeigen, dass die Gabe von entfärbtem Pandanusextrakt in der Lage ist die Zunahme des Körpergewichtes zu reduzieren.

**Beispiele**

**Beispiel 1:**

Gewinnung von kaltgepreßtem Öl

Die rote, kernhaltige Fruchtschicht wurde mit Zusatz von Wasser in einem Mixer gerührt um das rote Fruchtfleisch von den Kernen zu trennen. Der gewonnene, rote Fruchtfleischbrei wurde gepresst. Nach Zentrifugation und Filtration gewinnt man ein klares, rotes, dickflüssiges Öl (kalt gepresstes Virgin-Öl; Extrakt 1).

**Beispiel 2:**

Gewinnung des Öls durch eine Extraktion mit organischen Lösungsmitteln

Der wie oben gewonnene, rote Fruchtfleischbrei wird mit einem lipophilen Lösungsmittel, hier Pentan extrahiert. Alternativ könnten auch Hexan, Cyclohexan, Petrolether, Heptan, tert-Butylmethylether verwendet werden.

Nach Trocknung (Wasserentzug), Zentrifugation und Filtration erhält man das rote, dickflüssige Öl.

**Beispiel 3:**

Gewinnung der Alkoholextrakte

Der wie oben gewonnene, rote Fruchtfleischbrei wurde mit einem polaren Lösungsmittel hier Methanol extrahiert. Alternativ könnte auch Alkohol, Ether, Aceton, THF, Essigsäureethylester und bevorzugt Ethanol verwendet werden. Nach dem Abdampfen des Lösungsmittels erhält man den alkoholischen Extrakt.

**Beispiel 4:**

Gewinnung des Extraktes aus der getrockneten roten Fruchtschicht samt den Kernen durch eine Extraktion mit organischen Lösungsmitteln

Der rote Fruchtanteil samt den Kernen wurde bei unter 40°C im Trockenschrank getrocknet. Der Wassergehalt des getrockneten Materials lag bei unter 12%. 300g des trockenen Materials wurden zerkleinert und mit 1 L eines organischen Lösungsmittels hier Ethanol extrahiert. Man erhält ein dunkelrotes, dickflüssiges, öliges Produkt (Oleosum).

Solch eine Extraktion könnte selbstverständlich auch mit frischem Fruchtmaterial anstelle des getrockneten Fruchtfleisches durchgeführt werden.

Das Verhältnis des eingesetzten Fruchtmaterials zu dem gewonnenen Extrakt (DER) betrugt 1-6:1. Das optimale Verhältnis beträgt 3:1.

Das zuvor beschriebene Verhältnis ("Drug extract ratio"; DER) bezeichnet das Verhältnis aus der Menge an pflanzlichem Material eingesetzt zur Gewinnung eines Pflanzenpräparats und der Menge an erhaltenem Pflanzenpräparat.

**Beispiel 5:**

Extraktgewinnung aus dem roten Fruchtfleisch mit überkritischem Kohlendioxid (Tk = 31°C, Pk = 73,9 bar)

Im Extraktionsbehälter wurde der rote Fruchtbrei mit dem flüssigen Kohlendioxid versetzt (90 bar, 8 kg $CO_2$/h über 4 h). Das mit Pflanzeninhaltsstoffen beladene Lösungsmittel wurde mit Hilfe einer Pumpe in einen Abscheidebehälter gepumpt. Auf dem Weg dorthin wurde der Druck mittels eines Drosselventils auf unterkritische Bedingungen entspannt. Die Löslichkeit nimmt ab, und die gelösten lipophilen Stoffe fallen aus. Im Abscheidebehälter wurden die lipophilen Stoffe

**EP 2 429 561 B1**

(Ölfraktion) vom Gas getrennt. Das Gas wurde zum Fluid rekomprimiert und wieder verwendet. Durch Zugabe verschiedener Konzentrationen von Schleppmitteln (z.B. Methanol oder Ethanol) könnten auch mittelpolare und auch polare Substanzen schonend extrahiert werden.

**Beispiel 6:**

**Entfärbung des Pandanus-Extraktes durch Bestrahlung mit einem UVB-Strahler**

<u>Durchführung</u>

10 g Extrakt gewonnen nach Beispiel 4 wurden in einen 250 ml Rundkolben eingewogen. Der Rundkolben wurde in den Rotationsverdampfer eingespannt, in das Wasserbad abgesenkt und mit einem starken UVB-Strahler bestrahlt.
Lampe: Osram (300 W Ultra-Vitatux, 230 V - E 27/ES)
Abstand zum Kolben: ca. 20 cm.
Rotationsgeschwindigkeit: 170 Upm.
Das Wasserbad dient zum Temperaturausgleich und wird mit Zugabe von Eis bei einer Temperatur von 18 - 25 °C gehalten. Es wird nicht mit Vakuum gearbeitet. Nach bestimmten Behandlungszeiten wurden Proben zur Bestimmung der Farbintensität entnommen. Die Farbintensität wurde bei 478 nm bestimmt.

Tabelle 2: Gehalt an Farbstoffen nach der Bestrahlung:

|  | Dauer der Bestrahlung | Rest-Farbintensität im Vergleich zu der Farbintensität vor der Behandlung (%) (photometrische Bestimmung, bei 478 nm) |
|---|---|---|
| Pandanusextrakt | 16,5 Stunden | 45,8% |
| Pandanusextrakt | 39 Stunden | 0,7% |

**Beispiel 7:**

**Entfärbung des Pandanus-Extraktes durch Hydrierung**

2 g Extrakt gewonnen nach Beispiel 3 wurden in 10 ml Ethanol gelöst. Wasserstoff wurde in die alkoholische Lösung für 24 Stunden eingeleitet. Die Temperatur der Lösung wurde während der Behandlung konstant bei 40 °C gehalten. Nach 24 Stunden Behandlung konnte eine Farbintensitätsabnahme von 27 % beobachtet werden. Die Abnahme der Farbintensität durch Wasserstoffzufuhr konnte durch Zugabe von Katalysatoren $PtO_2$ und 1 % $Pd-CaCO_3$ wesentlich beschleunigt werden.

**Beispiel 8:**

**Entfärbung des Pandanus-Extraktes durch Oxidation mit Peroxiden**

2 g Extrakt gewonnen nach Beispiel 4 wurde in 10 ml Ethanol gelöst. Eine wässrige Lösung von Benzoylperoxid (Novadelox) in einer Endkonzentration von 0,001% wurde zugegeben. Die Lösung wurde bei 40°C (Wasserbad) 8 Stunden lang gerührt. Anschließend wurden das Ethanol und Wasser am Rotationsverdampfer abgedampft . Die Farbintensität des so behandelten Extraktes wurde dann bei 478 nm bestimmt. Eine Farbintensitätsabnahme von 84 % konnte erreicht werden.

**Beispiel 9:**

**Entfärbung des Pandanus-Extraktes durch Oxidation mit Peroxidase**

1g Extrakt gewonnen nach Beispiel 4 wurde in 5 ml Ethanol gelöst. 100 µL dieser Lösung wurden in 0,9 ml PBS pH 7,4 gegeben. Dieser Lösung wurde $H_2O_2$ in einer Endkonzentration von 1%(V/V) zugesetzt. Eine wässrige Lösung von Meerrettich Peroxidase (EC 1.11.1.7) in einer Endkonzentration von 1U/ml wurde zugegeben. Dieser Ansatz wurde bei Raumtemperatur 1 Stunde lang inkubiert. Anschließend wurden das Ethanol und Wasser am Rotationsverdampfer abgedampft . Die Farbintensität des so behandelten Extraktes wurde dann bei 478 nm bestimmt. Eine Farbintensitätsabnahme von 46 % konnte erreicht werden.

**Beispiel 10:**

**Antitumorale Aktivität des entfärbtes Extraktes von Pandanus conoideus an humanen Calu-6 Lungenkrebszellen**

Der Pandanusextrakt gemäß Beispiel 4 wurde wie unter Beispiel 6 beschrieben durch UVB-Bestrahlung entfärbt (entfärbter Extrakt A, Rest-Farbintensität < 1 %), wie unter Beispiel 7 beschrieben mittels Wasserstoff entfärbt (entfärbter Extrakt B, Restfarbintensität 73%) und wie unter Beispiel 8 beschrieben mittels Peroxiden entfärbt (entfärbter Extrakt C, Restfarbintensität 16%).

Die entfärbten Extrakte A bis C wurden daraufhin in folgendem Zellkulturassay getestet.

Zellkultur

Die humanen Calu-6 Lungenkrebs-Zellen wurden in 50 ml Zellkulturflaschen (Greiner) in einem RPMI 1640 Medium (Sigma, München), dem je 50 ml Zellkultur 10% fötales Kälberserum (Sigma, München) zugesetzt wurden, bei 37°C in feuchter, 5%iger $CO_2$ Atmosphäre aufbewahrt und 1 bis 2 mal wöchentlich mit frischem Medium versehen.

Protokoll für den Cytotoxizitätstest

Humane Calu-6 Lungenkrebs-Zellen wurde in 96-Lochplatten (Greiner) dispensiert (2 x 106 cell/plate).

Die Testextrakte A, B und C mit einer Restfarbintensität von einer Restfarbintensität von < 1 % entfärbter Extrakt A, einer Restfarbintensität von 73% entfärbter Extrakt B, einer Restfarbintensität von 16% entfärbter Extrakt C, im Vergleich zum unentfärbten Referenzextrakt gewonnen gemäß Beispiel 4 (unentfärbter Extrakt R) mit einer Farbintensität von 100% wurden entweder in Wasser oder Aceton mit einer Konzentration bis zu 10 mg/ml gelöst.

Die Aktivitäten der Extrakte wurden quantitativ bestimmt und als IC-50-Werte berechnet.

Alle Assays wurden dreimal ausgeführt. Nach Ablauf von drei Tagen gerechnet vom Beginn der Behandlung wurden die Zellen mittels 3%iger Formaldehydlösung fixiert und das Medium dekantiert. Die Zellen wurden mit Kristallviolett gefärbt. Die optische Dichte bei 595 nm (OD595) wurde mittels eines Multiscan Ascent ELISA Readers bestimmt.

Ergebnisse:

Die Aktivitäten des unbehandelten Extraktes (unentfärbter Extrakt R) und der entfärbten Extrakte A, B, C unterscheiden sich überraschenderweise wenig (obwohl eine Reduktion der Farbintensität bis fast 100% durch die Behandlung erreicht werden konnte. Die Ergebnisse deuten darauf hin, dass die Farbstoffe offenbar für die Antikrebs-Wirkung des Extraktes wenig Bedeutung haben.

Tabelle 3:

| Extrakt | A | B | C | R |
|---|---|---|---|---|
| IC50 [μg/ml] | 44.17 | 41.32 | 46.81 | 40.12 |

**Beispiel 11:**

**Unterdrückung des proinflammatorischen Cytokins IL-6 durch die entfärbten Pandanus-Extrakte**

Methode:

Ein gerinnungsgehemmtes Voll-Blut wird in Zellkulturplatten mit LPS (Lipopolysaccacharide, 10 ng/ml) versetzt und für 24 h bei 37°C im Zellkultur-Brutschrank inkubiert. Die Extrakte gemäß Beispiel 10 (10 μg/ml unentfärbter Extrakt R mit 100% Farbintensität oder entfärbte Extrakte A, B, C mit < 1%, 73%, 16% Restfarbintensität) werden jeweils 15 Minuten vor der LPS-Stimulation zugegeben. Nach 24 Stunden werden die Überstände abgetrennt, zentrifugiert und zur Bestimmung von IL-6 Konzentrationen mit ELISA verwendet.

Ergebnisse

Die Tabelle 4 zeigt, dass die Unterschiede in der Unterdrückung der IL-6-Konzentration durch die Extrakte A, B, C und R relativ gering ausfallen, obwohl die Farbstoff-Konzentrationen in den Extrakten sich stark unterscheiden. Es gibt also wenig Korrelation zwischen der Farbintensität der Extrakte und der antientzündlichen Wirkung.

Dies ist ein Indiz, dass die antiinflammatorische Wirkung des Pandanus-Extraktes vermutlich nicht auf den Farbstoffen des Extraktes basiert.

Tabelle 4: Unterdrückung des proinflammatorischen Cytokins IL-6 durch die entfärbten und den unentfärbten Pandanus-Extrakt

| Proben | IL-6 Konzentration in % (pg/ml) |
|---|---|
| nicht stimuliert | 0 |
| stimuliert mit LPS (10 ng/ml) | 100 % (= 52400 pg IL-6/ml) |
| Stimulation mit LPS 10 ng/ml + 10 μg/ml unbehandelten Pandanusextrakt R (100% Farbintensität) | 38% |
| Stimulation mit LPS 10 ng/ml + 10 μg/ml Pandanusextrakt A | 40% |
| Stimulation mit LPS 10 ng/ml + 10 μg/ml Pandanusextrakt B | 48% |
| Stimulation mit LPS 10 ng/ml + 10 μg/ml Pandanusextrakt C | 49% |

Tabelle 5: Biologische Aktivitäten (antitumorale und antientzündliche Wirkung) der entfärbten Extrakte mit unterschiedlichen Farbintensitäten.

| Farbintensität-Abnahme in % | Biologische Aktivität | |
|---|---|---|
| | Veränderung der Antiinflammationswirkung* (IL-6 Suppression) | Veränderung der Cytostatischen Wirkung** (IC-50-Werte, Calu-6 Krebszellen) |
| - 99 % (UV, 25 °C) Extrakt A | -5% | -10% |
| - 84 % (Benzoylperoxyd) Extrakt C | -29% | -15 % |
| -27% (PtO$_2$, Hydrierung) Extrakt B | -26% | -3% |

*) Die Veränderungen der antiinflammatorischen Wirkungen wurden aufgrund der Veränderungen der IL-6 Konzentrationen im Vergleich zum Referenzextrakt R bestimmt (siehe auch Tab. 4).
**) Die Veränderungen der antitumoralen Aktivitäten wurden aufgrund der Veränderungen der IC50-Werte im Vergleich zum nicht entfärben Referenzextrakt R bestimmt (s. auch Beispiel 10).

**Beispiel 12:**

Herstellung topischer Formulierungen

Formulierung 1:

| Nr. | Bestandteile | Anteil (Gew.-%) |
|---|---|---|
| 1 | Lecithin | 4,0 |
| 2 | Extrakt A | 5,0 |
| 3 | Glycerin | 5,0 |
| 4 | Propylenglykol | 16,0 |
| 5 | Mittelkettige Triglyceride | 3,0 |
| 6 | Wollwachs | 10,0 |
| 7 | gereinigtes Wasser | ad 100 |

Formulierung 2:

| Nr. | Bestandteile | Anteil (Gew.-%) |
|---|---|---|
| 1 | Extrakt C | 6,0 |
| 2 | Zinkoxid | 10,0 |
| 3 | Glycerin | 6,0 |
| 4 | Dickflüssiges Paraffin | 30,0 |
| 5 | Weißes Vaselin | 20,0 |
| 6 | Gebleichtes Wachs | ad 100 |

Formulierung 3:

| Nr. | Bestandteile | Anteil (Gew.-%) |
|---|---|---|
| 1 | Extrakt C | 2,0 |
| 2 | Dexpanthenol | 1,5 |
| 3 | Miglyol | 5,0 |
| 4 | gereinigtes Wasser | 5,0 |
| 5 | Basiscreme DAC | Ad 100 |

Formulierung 4:

| Nr. | Bestandteile | Anteil (Gew.-%) |
|---|---|---|
| 1 | Extrakt C | 3,0 |
| 2 | Mittelkettige Triglyceride | 5,0 |
| 3 | Polysorbat 60 | 8,0 |
| 4 | Propylenglycol | 6,0 |
| 5 | Erdnussöl | 10,0 |
| 6 | Glycerin | 10,0 |
| 7 | Vaselinum album | ad 100 |

Formulierung 5:

| Nr. | Bestandteile | Anteil (Gew.-%) |
|---|---|---|
| 1 | Extrakt B | 10,0 |
| 2 | Harnstoff | 4,0 |
| 3 | Milchsäure | 1,0 |
| 4 | Kaliumsorbat | 0,15 |
| 5 | gereinigtes Wasser | 35,0 |
| 6 | Wollwachsalkoholsalbe | ad 100 |

Formulierung 6:

| Nr. | Bestandteile | Anteil (Gew.-%) |
|---|---|---|
| 1 | Extrakt A und Extrakt B (50:50) | 8,0 |
| 2 | Zinkoxid | 15,0 |
| 3 | Talkum | 15,0 |
| 4 | Glycerol | 30,0 |
| 5 | Propylenglycol | 10,0 |
| 6 | qereiniqtes Wasser | ad 100 |

Formulierung 7, Hautgel:

| Nr. | Bestandteile | Anteil (Gew.-%) |
|---|---|---|
| 1 | Extrakt A | 8,0 |
| 2 | Natriumhydroxidlösung | 6,0 |
| 3 | Propandiol-1,2 | 15,0 |
| 4 | Glycerol | 3,0 |
| 5 | Polyacrylsäure | 10,0 |
| 6 | gereinigtes Wasser | ad 100 |

Alle Bestandteile wurden vermischt und bis zur Homogenität gerührt und auf 60°C für 10 Minuten unter Rühren erhitzt. Die Lösung wurde dann, ebenfalls unter Rühren, auf Zimmertemperatur abgekühlt. Während dieser Phase bildet sich ab ungefähr 45°C ein Gel.

Formulierung 8 (Gel):

| Nr. | Bestandteile | Anteil (Gew.-%) |
|---|---|---|
| 1 | Extrakt C | 4,0 |
| 2 | Acrylat/C10-30 Alkylacrylat Crosspolymer | 0,2 |
| 3 | Polyacrylsäure | 0,10 |
| 4 | Xanthan Gummi | 3,0 |
| 5 | Cetearylalkohol | 4,0 |
| 6 | C12-15 Alkylbenzoat | 3,0 |
| 7 | Caprylic/Capric Triglycerid | 5,0 |
| 8 | Zyklischer Dimethylpolysiloxan | 1,0 |
| 9 | Isopropanol | 3,0 |
| 10 | Natriumhydroxid | q.s. |
| 11 | Wasser | ad 100 |

Formulierung (Wasserfrei Formulierung) 9:

| Nr. | Bestandteile | Anteil (Gew.-%) |
|---|---|---|
| 1 | Extrakt A | 4,0 |
| 2 | Polygceryl-3-Diisostearate | 3,5 |
| 3 | Glycerin | 14,0 |

(fortgesetzt)

| Nr. | Bestandteile | Anteil (Gew.-%) |
|---|---|---|
| 4 | Polyglyceryl-2-Dipolyhydroxystearate | 3,5 |
| 5 | Miglyol | 6,0 |
| 6 | Proppylenglykol | 2,0 |
| 7 | Sonnenblumenöl | 4,0 |
| 8 | Vaselinum album | ad 100 |

Formulierung (Creme) 10:

| Nr. | Bestandteile | Anteil (Gew.-%) |
|---|---|---|
| 1 | Extrakt A | 5,0 |
| 2 | Glycerylstearat | 2,5 |
| 3 | Stearylalkohol | 2,0 |
| 4 | PEG-40-Stearat | 1,0 |
| 5 | Cetylalkohol | 2,0 |
| 6 | SHEABUTTER | 2,0 |
| 7 | Carrageen | 3,0 |
| 8 | Tapiokastärke | 1,0 |
| 9 | Ethylhexylmethoxycinnamat | 2,0 |
| 10 | Phenoxyethanol | 0,4 |
| 11 | $C_{12}$-$_{15}$Alkylbenzoat | 3,0 |
| 12 | p-Hydroxybenzoesäurealkylester (Paraben) | 0,4 |
| 13 | Aristoflex AVC (Ammonium PolyacryldimethyltauridNinylformamid-copolymer) | 0,2 |
| 14 | 2-Isopropyl-5-Methyl-Cyclohexancarbonyl-D-Alaninethylexter | 0,5 |
| 15 | Glycerin | 8,0 |
| 16 | EDTA | 0,2 |
| 17 | Wasser | ad 100 |

**Beispiel 13:**

Herstellung eines trinkfertigen Granulats zur Appetithemmung

| | |
|---|---|
| Extrakt A | 15 g |
| Mikrokristalline Cellulose | 25 g |
| Aerosil | 8 g |
| Walocel CRT | 8 g |
| Magnesiumstearat | 4 g |
| Puderzucker | 28 g |
| Ethanol ad | 100 g |

Geschmacksstoffe und Aromastoffe wie Orangengeschmack, Maracujageschmack können zugesetzt werden.

Alle Bestandteile wurden vermischt und bis zur Homogenität gerührt und getrocknet. Das Granulat wurde durch ein Sieb gegeben. Zur Herstellung einer Trink-Lösung wurden 5 g des Granulats in 100 ml Wasser eingerührt. Die wässrige Suspension ist trinkfertig. Selbstverständlich kann das Granulat auch in Joghurt oder Milch eingerührt und eingenommen werden.

**Beispiel 14:**

Herstellung eines magensaftresistenten Granulats mit verzögerter Freisetzung zur Appetithemmung

Herstellung der Pellets:

| | |
|---|---|
| Extrakt A | 10 g |
| Aerosil | 10 g |
| Lactose | 120 g |

Die Binderlösung besteht aus 7%iger Polyvinylpyrrolidon-Lösung in 600 ml Wasser. Die Zuckerkerne werden mit der Binderlösung befeuchtet und die Mischung von Pandanusextrakt B / Aerosil wird auf die Pellets aufgetragen. Das Granulat wird getrocknet und gesiebt. Man erhält Pellets mit Körnchengröße von 0,5-1 mm. Die Pellets werden nun mit Eudragit beschichtet.

Überzug der Pellets mit Eudragit L 30:

| | |
|---|---|
| Pellets | 100 g |
| Eudragit L 30 | 60 g |
| Talkum | 10 g |
| Triethylcitrat | 2 g |
| Wasser | 50 g |

Talkum und Triethylcitrat werden 20 Minuten lang im Wasser homogenisiert. Die Suspension wird in die Eudragit-Dispersion unter Rühren gegeben. Die Dispersion wird gerührt bis der Sprühvorgang abgeschlossen ist. Die Pellets werden in Sachet zu 5-10 g pro Portion abgefüllt. Die Pellets können mit Joghurt oder Fruchtsäften vermischt bzw. suspendiert und so eingenommen werden.

**Beispiel 15:**

Herstellung von Pandanusextrakt B in Soft-Gelatine-Kapseln

Der Extrakt kann in einer Menge von 300 mg bis 1000 mg in Soft-GelatineKapseln konfektioniert werden.

**Beispiel 16:**

Herstellung von Fitness-Riegeln zur Gewichtskontrolle

| | |
|---|---|
| Extrakt A | 50 g |
| Haferflocken | 100 g |
| Proteinpulver | 50 g |
| Eiweiss/Eiklar | 50 g |
| Milch | 200 g |
| Rosinen | 20 g |

Die Bestandteile werden vermischt und bis zur Homogenität gerührt, in die Form gegossen und bei 180°C für ca. 30 Minuten im Backofen erhitzt. Es ergeben sich 10 Riegel-Stücke, jedes Riegelstück enthält 5 g Pandanusextrakt B.

Beispiel 17:

Herstellung einer Injektionslösung

| | |
|---|---|
| Extrakt A | 10 mg |
| Ethanol | 20 Vol % |
| Propylenglykol | 100 mg |
| Wasser für Injektionszwecke | ad 1 mL |

Natriumhydroxid wurde zur pH-Wert Einstellung auf physiologische Werte (pH7,4) genutzt.

**Beispiel 18:**

Durchführung eines Tierversuchs an Ratten

Wistar-Ratten wurden mit Extrakt A 250 mg / kg Körpergewicht 7 Tage lang behandelt (n= 6). Die orale Applikation des Extraktes A wurde täglich mittels einer Sonde verabreicht. Die Tiere in der Kontrollgruppe wurden 5 Tage mit Palmöl behandelt. In der Tab. 6 ist eindeutig zu sehen, dass der Durchschnitt des Körpergewichts der Tiere in der Pandanusextrakt A-Gruppe in dem Behandlungszeitraum sich nur geringfügig verändert (Tag 0 = 178 g, Tag 7 = 182 g), während der Durchschnitt des Körpergewichts der Tiere in der Kontrollgruppe stetig von 159 g am Tag 0 bis zu 185 g am Tag 5 anstieg (Tab.6).

Es konnten keine signifikanten Veränderungen der relativen Lebergewichte (des Verhältnisses von Leber- zu Körpergewicht) und der Leberenzym-Parameter (SGOT, SGPT, alkalische Phosphatase) festgestellt werden. Dies deutet auf die gute Verträglichkeit und die Sicherheit für die Anwendung des Extraktes hin.

Tabelle 6. Einfluss von entfärbtem Pandanusextrakt A auf das Körpergewicht bei Ratten (n= 6); $\sigma$ = Standardabweichung

| Kontrollgruppe (Palmöl) | Tag 0 | Tag 1 | Tag 2 | Tag 3 | Tag 4 | Tag 5 | | |
|---|---|---|---|---|---|---|---|---|
| Mittelwert (g) | 160,0 | 166,8 | 174,0 | 178,9 | 180,6 | 185,8 | | |
| $\sigma$ | 18,7 | 12,6 | 16,5 | 17,3 | 16,7 | 18,2 | | |
| | | | | | | | | |
| **Pandanus 250 mg / kg KG** | **Tag 0** | **Tag 1** | **Tag 2** | **Tag 3** | **Tag 4** | **Tag 5** | **Tag 6** | **Tag 7** |
| **Mittelwert (g)** | 177,8 | 174,4 | 185,0 | 187,9 | 189,6 | 192,3 | 182,00 | 181,8 |
| $\sigma$ | 6,2 | 3,2 | 4,8 | 5,7 | 5,1 | 5,6 | 4,9 | 7,0 |

**Beispiel 19: klinische Untersuchungen**

PRINZIP UND METHODIK:

Ziel dieser Untersuchung war es, einen erfindungsgemäßen Extrakt auf seine Verträglichkeit und Wirksamkeit nach klinisch-dermatologischen Prüfkriterien zu untersuchen. Die Patienten konnten täglich den testbegleitenden Dermatologen bei objektiven und subjektiven Hautveränderungen zu Rate ziehen.

Zu Beginn der Studie, nach 2 Wochen Anwendung sowie nach 3 Wochen Anwendung, erfolgten die dermatologischen Untersuchungen der Studienteilnehmer mit Erhebung des modifizierten SCORAD's.

Jede Testperson wandte anschließend den erfindungsgemäßen Extrakt zweimal täglich insgesamt drei Wochen lang auf den betroffenen Hautstellen an. Bei Bedarf konnten tägliche Kontrollen des Hautzustandes durch den testbegleitenden Dermatologen durchgeführt werden.

Der verwendete erfindungsgemäße Extrakt:

Der in der klinischen Studie eingesetzte erfindungsgemäße Extrakt wurde gemäß Extrakt A aus Beispiel 11 hergestellt und als Creme mit einer Konzentration von 3% Pandanusextrakt formuliert. Die Kontrollgruppe wurde mit einer Creme behandelt, die bis auf den Pandanusextrakt die gleiche Zusammensetzung aufwies. In dieser Creme der Kontrollgruppe 3% nicht entfärbter Pandanusextrakt gewonnen nach Beispiel 4 enthalten.

**Tab.** 7 Zusammensetzung der angewendeten Nanoemulsion-Creme für die Anwendungsstudie bei Neurodermitikern - A entfärbter Extrakt - Zusammensetzung der Creme

|  | % |
|---|---|
| Dipalmitoyl-Phosphatidylcholin | 5,000 |
| Glycerol 85% | 1,500 |
| Dexpanthenol | 1,000 |
| Urea | 3,000 |
| Pandanusextrakt entfärbt (Extrakt A, Beispiel 11) | 3,000 |
| Tocopherolacetat | 0,200 |
| Sodium benzoate | 0,150 |
| Aq. Dest. | 86,15 |
|  | 100,00 |

- B farbiger Extrakt - Zusammensetzung der Creme

|  | % |
|---|---|
| Dipalmitoyl-Phosphatidylcholin | 5,000 |
| Glycerol 85% | 1,500 |
| Dexpanthenol | 1,000 |
| Urea | 3,000 |
| Pandanusextrakt (Extrakt nach Beispiel 4) | 3,000 |
| Tocopherolacetat | 0,200 |
| Sodium benzoate | 0,150 |
| Aq. Dest. | 86,15 |
|  | 100,00 |

Patienten:

Das Patientenkollektiv bestand aus erwachsenen Testpersonen mit Hautveränderungen der Neurodermitis, i.S. Juckreiz, Rötung, Schuppung oder Trockenheit. Untersucht wurden 20 Personen mit leichter bis mittelstarker Neurodermitis. Je Zusammensetzung der Creme wurden 10 Personen behandelt.

| Nr. | Geschlecht | Alter | Diagnose | Applikationsort |
|---|---|---|---|---|
| 1. | w | 65 | Neurodermitis | Unterschenkel |
| 2. | w | 52 | Neurodermitis | Oberarm |
| 3. | w | 57 | Neurodermitis | Oberkörper |
| 4. | w | 52 | Neurodermitis | Oberkörper |
| 5. | w | 30 | Neurodermitis | Ellenbeuge |
| 6. | w | 36 | Neurodermitis | Oberkörper |
| 7. | w | 44 | Neurodermitis | Oberkörper |
| 8. | m | 45 | Neurodermitis | Bein |
| 9. | m | 42 | Neurodermitis | Unterarme |

(fortgesetzt)

| Nr. | Geschlec ht | Alter | Diagnose | Applikationsort |
|---|---|---|---|---|
| 10. | w | 67 | Neurodermitis | Kniekehle, Fußsohle |
| 11. | w | 40 | Neurodermitis | Oberkörper |
| 12. | w | 55 | Neurodermitis | Ellenbeuge |
| 13. | m | 45 | Neurodermitis | Oberkörper |
| 14. | m | 48 | Neurodermitis | Ellenbeuge |
| 15. | w | 73 | Neurodermitis | Kniekehle |
| 16. | m | 37 | Neurodermitis | Unterarme |
| 17. | w | 31 | Neurodermitis | Kniekehle |
| 18. | m | 53 | Neurodermitis | Ellenbeuge |
| 19. | w | 70 | Neurodermitis | Oberarme |
| 20. | w | 68 | Neurodermitis | Unterschenkel |

Ausschlusskriterien:

- Akute organische Krankheit
- Schwangerschaft und Stillzeit
- Bestehende Sensibilisierung auf Inhaltsstoffe der Prüfprodukte
- Schwerwiegende Erkrankungen
- Applikation von wirkstoffhaltigen Präparaten und Pflegemitteln bis 4 Wochen vor Testbeginn
- Einnahme von Medikamenten gegen Neurodermitis/ Atopie (Glucocorticoide, Antiallergika, topische Immunmodulatoren, etc.)

Durchführung:

Es wurden nur Teilnehmerinnen und Teilnehmer in die Prüfgruppen aufgenommen, bei denen sich keinerlei sonstige behandlungsbedürftige Hautveränderungen fanden.

Zu Beginn der Studie, nach 2 Wochen Anwendung sowie nach 3 Wochen Anwendung, erfolgte eine dermatologische Untersuchung der Studienteilnehmer mit Erhebung des ärztlichen Beurteilungsbogens.

Der erfindungsgemäße Extrakt wurde den Probanden mit der Applikationsinstruktion über mindestens 2x-täglicher Applikation ausgehändigt. Weiterhin wurden die Probanden instruiert, in der Zeit des Anwendungstests auf andere Präparate zu verzichten.

Atopie-Score (modifiziert nach SCORAD):

Dieser Index dient der qualitativen und quantitativen Einschätzung des Schweregrades des atopischen Ekzems, modifizierter SCORAD (= Severity Scoring of Atopic Dermatitis).

Die standardisierte Beurteilung erfolgt durch die Angabe :

A) des Ausprägungsgrades sechs typischer morphologischer Veränderungen (Grad 0-3, Gesamtwert hier: max 10)
B) des Anteils der betroffenen Hautfläche (%) bzgl. Rötung / Kruste / Excoriation
C) der subjektiven Einschätzung von Juckreiz anhand einer visuellen Analogscala (Grad 0-10; hier: max. 7), außerdem des nächtlichen Schlafverlustes (Grad 0-10, hier: max 3).

zu A)

Klassifiziert werden sechs typische morphologische Veränderungen:

Hautrötung

Krusten

flächenhafte Infiltration d. Haut mit Vergröberungen der Hautfelder

Schwellung/Bläschen (hier: Ausschlußkriterium)

Hautabschürfung bis ins Korium (hier: Ausschlußkriterium)

Trockenheit (wird an den NICHT betroffenen Hautstellen erhoben).

Kriterium: nicht vorhanden = 0 / milde vorhanden = 1 / mittelgradig = 2 / ausgeprägt = 3

Die Summe der Werte kann zwischen 0-10 schwanken, wobei die Ausprägung von Ödem oder Papeln als Ausschlußkriterium definiert wurde, als Anhalt für einen akuten neurodermitischen Hautzustand mit medizinischer Behandlungsbedürftigkeit. Hautabschürfung bis ins Korium wurde als Ausschlußkriterium im Rahmen der Eingangsuntersuchung definiert. Exkoriationen im Verlauf der Studie waren Indikation für eine Testunterbrechung von 2 Tagen.

Zu B)

Überschreitet der Gesamtwert des SCORAD 50 aufgrund einer großflächigen Ausprägung führt dies ebenfalls zum Ausschluß des Probanden.

Zu C)

Die Einschätzung des Juckreizes >7 und des Schlafverlustes >3 führt ebenfalls zum Ausschluß, da medizinische Behandlungsbedürftigkeit, evtl. Artefakte anzunehmen sind.

Der maximale Gesamtwert als modifizierter SCORAD nach Dermatest ist 50, als Ausdruck einer mittleren Ausprägung des atopischen Krankheitsbildes. Berechnung des Index: 7A/2+B/5+C.

Dermatologische Untersuchungen:

1. Vor Beginn des Anwendungstests

Alle 20 Studienteilnehmer zeigten entweder leichte Rötung, Schuppung oder Trockenheit. Weitere pathologische Hautveränderungen waren in keiner Form festzustellen.

2. Während des Anwendungstests

Keine der 20 Studienteilnehmer zeige im Verlauf des dreiwöchigen Anwendungstests pathologische Hautveränderungen. Testunterbrechungen oder gar hautfachärztliche Behandlungen waren nicht notwendig.

3. Nach Ende des Anwendungstests

Bei der dermatologischen Abschlussuntersuchung nach dem Testende zeigten sich bei den 20 Studienteilnehmern keine zusätzlichen pathologische Hautveränderungen. Der erfindungsgemäße Extrakt wurde sehr gut vertragen und führte bei keiner Testperson zu unerwünschten Hautveränderungen.

Dermatologische Beurteilungskriterien erfaßt durch mod. SCORAD:

| 1. Ödem/Papeln | 5. Lichenifikation |
|---|---|
| 2. Rötung/Erythem | 6. Trockenheit (Xerosis) |
| 3. Krustenbildung | 7. Verstärkter Juckreiz |
| 4. Excoriation | 8. Schlafverlust |

Subjektive Beurteilungskriterien:

1. Schweregrad der Erkrankung

2. Rötung/Erythem

3. Trockenheit

4. Juckreiz

5. Wirkung des Prüfpräparates

Die Bewertung erfolgt durch die Probanden im Rahmen der täglichen Dokumentation im Probandentagebuch. Die Skala umfaßt den Bereich 0-7, 0 = nicht vorhanden, 7= sehr stark vorhanden.

BEURTEILUNG DER TESTERGEBNISSE:

Insgesamt 10 Studienteilnehmer vertrugen im insgesamt dreiwöchigen Anwendungstest nach dermatologisch-klinischen Kriterien den erfindungsgemäßen Extrakt einwandfrei. Es kam in keinem Fall zu unerwünschten oder gar pathologischen Hautveränderungen. Nach der Anwendung des erfindungsgemäßen Extraktes über 14 Tage ergab sich eine Reduktion des durchschnittlichen SCORAD-Wertes von 32,01 auf 19,31. Dies entspricht einer Verminderung um -39,68 % zum Ausgangswert. Nach der Anwendung des nicht entfärbten Extraktes über 14 Tage ergab sich eine Reduktion des durchschnittlichen SCORAD-Wertes von 31,65 auf 19,87. Dies entspricht einer Verminderung um -37,21 % zum Ausgangswert.

Nach der Anwendung des erfindungsgemäßen Extraktes über 21 Tage ergab sich bei den Studienteilnehmern eine Reduktion des durchschnittlichen SCORAD-Wertes von 32,01 auf 7,2. Dies entspricht einer Verminderung der Symptome um -77,5 % zum Ausgangswert. Nach der Anwendung des nicht entfärbten Extraktes über 21 Tage ergab sich bei den Studienteilnehmern eine Reduktion des durchschnittlichen SCORAD-Wertes von 31,65 auf 6,6. Dies entspricht einer Verminderung der Symptome um -79,1 % zum Ausgangswert.

Der Rückgang des Atopie-Index ist daher als exzellent zu bewerten und unterscheidet sich zwischen der Anwendung des entfärbten und nicht entfärbten Extraktes nur geringfügig. Mit der Verwendung des erfindungsgemäßen, entfärbten Extrakts konnte also ein annähernd gleich guter Behandlungserfolg erzielt werden.

**Beispiel 20: Erfahrungsbericht mit Pandanus-Extrakt-Nanoemulsionen ohne hautpflegende Zusatzstoffe**

Die Anwendung von Pandanus-Extrakt-Nanoemulsion wurde bei drei Probanden mit atopischem Ekzem und mit symmetrischen befallenen Stellen beobachtet. Die befallene Stelle wurde entweder mit Pflege-Lotion (freie Wahl) oder mit Pandanus-Nanoemulsion zweimal täglich behandelt (links-rechts-Vergleich). Die Behandlungs- und Beobachtungszeit war eine Woche. Die Veränderung der Hauptsymptomatik der Erkrankung, nämlich Juckreiz. wurde vor und nach der Behandlung und zwischen den Behandlungen verglichen. Alle 3 Probanden hatten vor Beginn der Behandlung ein starkes bis mittelstarkes Juckreiz-Leiden. (Abstufung der Juckreiz-Symptomatik: starke - mittelstarke - milde - keine Beschwerden)

**Tab.8** Zusammensetzung der Nanoemulsion nur mit Pandanusextrakt und Emulgatoren

|  | % |
| --- | --- |
| Dipalmitoyl-Phosphatidylcholin | 6,00 |
| Mygliol | 8,00 |
| Pandanusextrakt entfärbt (Extrakt A, Beispiel 11) | 3,00 |
| Sodium benzoate | 0,15 |
| Aq. dest. | 82,85 |
|  | 100,00 |

Nach 1 Woche Behandlung mit der Pandanus-Nanoemulsion berichteten die Probanden eine starke Abnahme des Juckreizes (keine Beschwerden : 2 Probanden, milde Beschwerden 1 Proband), wohingegen die Anwendung mit Pflege-Lotion weniger Erleichterung im Vergleich zu der Anwendung mit Pandanus-Creme zeigte (mittelstarke Beschwerden 2 Probanden/ starke Beschwerden: 1 Proband).

**Beispiel 21: Erfahrungsbericht mit Pandanusextrakt-Nanoemulsionen zur Behandlung gegen Psoriasis**

Die Anwendung von Pandanusextrakt-Nanoemulsionen zur Behandlung gegen Psoriasis wurde bei sechs Probanden mit symmetrisch befallenen Stellen beobachtet. Die sechs Probanden wendeten die Pandanusextrakt-Nanoemulsion A (entfärbt) und die Pandanusextrakt-Nanoemulsion B (nicht entfärbt) zweimal täglich über einen Zeitraum von drei Monaten an (Links-rechts-Vergleich). Als Beurteilungsparameter diente der Vergleich der von den Patienten berichteten Ausgangssymptome mit den Symptomen nach 3 monatiger Behandlung mit den Nanoemulsionen. Alle 6 Probanden hatten vor Beginn der Behandlung mittelstarke Rötung-Schuppung-Beschwerden (Abstifung der Symptomatik: Pandanusextrakt-Nanoemulsion B berichteten die Probanden ebenfalls von einer Abnahme der Symptomatik (mittelstark → mittelstark : 2 Probanden; mittelstark → milde Beschwerden: 4 Probanden)

Beide Pandanusextrakt-Nanoemulsionen zeigten also eine ähnlich erfolgreiche Behandlung von Patienten mit Psoriasis.

**Tab.9** Zusammensetzung der angewendeten NanoemulsionCreme zur **Behandlung von** Psoriasis

|  | % |
| --- | --- |
| Dipalmitoyl-Phosphatidylcholin | 5,000 |
| Glycerol 85% | 1,500 |
| Dexpanthenol | 1,000 |
| Urea | 3,000 |
| Pandanusextrakt | 3,000 |
| Tocopherolacetat | 0,200 |
| Sodium benzoate | 0,150 |
| Aq. Dest. | 86,15 |
|  | 100,00 |

**Beispiel 22: Bestimmung der Extinktion eines entfärbten Extraktes**

100 mg Extrakt gewonnen nach Beispiel 6 wurden in 100 ml Methanol gelöst und 10 min. beschallt und erneut 1 zu 2 in Methanol verdünnt. Die Endkonzentration betrug somit 0,5 mg/ml.

Zur Erstellung einer Standardstocklösung wurden 100mg nicht entfärbter Extrakt (nach Beispiel 4) in 100ml Methanol gelöst, 10 min. beschallt und ebenfalls nochmals verdünnt (0,2 mg/ml;).

Von allen gemessenen Extinktionen wurde der Leerwert (Methanol) subtrahiert. Mit Hilfe der folgenden Formel lässt sich die Restfarbintensität der entfärbten Extrakte berechnen.

$$\frac{A_{Probe}}{A_{Standard}} \times \frac{Wt_{Standard}}{100} \times \frac{100}{Wt_{Probe}} \times 100 = \% \ w/w$$

$A_{Probe}$: Extinktion der Probe (entfärbter Extrakt)
$A_{Standard}$: Extinktion des Standards (nicht entfärbter Extrakt)
$Wt_{Standard}$: Gewicht des Standards (nicht entfärbter Extrakt)
$Wt_{Probe}$: Gewicht der Probe (entfärbter Extrakt)

Die Extinktionswerte lagen bei $A_{Probe}$=0,133 und $A_{Standard}$=0,116. Damit ergibt sich eine Restfarbintensität der gemessenen Probe eines entfärbten Extraktes von 45,86%. Dies bedeutet die Entfärbung des Extraktes aus Beispiel 6 lag nach 16,5 Stunden UVB-Bestrahlung bei 54, 14%.

**Patentansprüche**

**1.** Verfahren zur Herstellung eines entfärbten Extraktes umfassend die folgenden Schritte:

I) Bereitstellung eines farbigen Extraktes aus der Frucht der Spezies Pandanus conoideus,
II) Behandlung des farbigen Extraktes mittels

a) Bestrahlung durch UV-Licht, oder
b) Oxidation mit Peroxiden, oder
c) Oxidation mit Sauerstoff, oder
d) Oxidation mit reduzierbaren Halogenverbindungen, oder
e) Hydrierung mit Wasserstoff und Hydrierungskatalysatoren oder

f) Biologischer Oxidation durch oxidative Enzyme.

2. Verfahren gemäß Anspruch 1, wobei es sich bei den reduzierbaren Halogenverbindungen um Chlorwasser, Chlor, Brom, Iod, HClO (hypochlorige Säure oder unterchlorige Säure), Hypochloride, $ClO_2$, $HClO_2$, Chlorite, $HClO_3$, Chlorate, $HClO_4$, Perchlorate.

3. Verfahren gemäß Anspruch 1 oder 2, wobei Verarbeitung des farbigen Extraktes bei Temperaturen unterhalb von 50°C durchgeführt wird.

4. Entfärbter Extrakt erhältlich nach einem Verfahren gemäß eines der Ansprüche 1 - 3.

5. Entfärbter Extrakt gemäß Anspruch 4 zur nichttherapeutischen Verwendung als Appetitzügler und Sattmacher und zur Verhinderung einer erneuten Gewichtszunahme nach absolvierter Diät.

6. Entfärbter Extrakt gemäß Anspruch 4 zur Verwendung bei der Behandlung von Übergewicht, Fettleibigkeit oder Fresssucht.

7. Entfärbter Extrakt gemäß Anspruch 4 zur Verwendung bei der Prophylaxe und/oder Behandlung von immuno-dermatologischen Erkrankungen, Tumoren, Krebserkrankungen, rheumatoiden Erkrankungen, chronisch-rezidiven Entzündungen des Darms, degenerativen Erkrankungen, neurodegenerativen Erkrankungen, Inflammationen, gastro-intestinalen Erkrankungen, Diabetes, kardiovaskulären Erkrankungen, immunologischen Erkrankungen, Infektionserkrankungen, ophthalmologischen und otologischen Erkrankungen.

8. Entfärbter Extrakt gemäß Anspruch 4 zur Verwendung nach Anspruch 7, wobei die immuno-dermatologischen Erkrankungen, Tumore, Krebserkrankungen, rheumatoiden Erkrankungen, chronisch-rezidiven Entzündungen des Darms, degenerativen Erkrankungen, neurodegenerativen Erkrankungen, Inflammationen, gastrointestinalen Erkrankungen, Diabetes, kardiovaskulären Erkrankungen, immunologischen Erkrankungen, Infektionserkrankungen, ophthalmologischen und otologischen Erkrankungen ausgewählt werden aus der Gruppe umfassend oder bestehend aus: Neurodermitis, Dermatitis, Ekzeme, Psoriasis, Arthritische Psoriasis, Rheumatoide Arthritis, Osteoarthritis, Stoffwechselstörungen der Haut, trockene Haut, Akne, aktinische Keratosis, akute und chronische myeloische Leukämie, akute und chronische lymphatische Leukämie, Adenokarzinome, Aderhautmelanom, Akustikusneurinom, Ampullenkarzinom, Analkarzinom, Astrozytome, Basaliom, Bauchspeicheldrüsenkrebs, Bindegewebstumor, Blasenkrebs, Bronchialkarzinom, kleinzelliges und nicht-kleinzelliges Bronchialkarzinom, Brustkrebs, Burkitt-Lymphom, Corpuskarzinom, CUP-Syndrom, Dickdarmkrebs, Dünndarmkrebs, Dünndarmtumore, Eierstockkrebs, Endometriumkarzinom, Ependymom, Epithel-Krebsarten, Ewing-Tumoren, gastrointestinale Tumore, Gallenblasenkrebs, Gallenkarzinome, Gebärmutterkrebs, Gebärmutterhalskrebs, Glioblastome, gynäkologische Tumore, Hals-, Nasen- und Ohrentumore, hämatologische Neoplasien, Haarzell-Leukämie, Harnröhrenkrebs, Hautkrebs, Hirntumore, Hirnmetastasen, Hodenkrebs, Hypophysentumor, Karzinoide, Kaposi-Sarkom, Kehlkopfkrebs, Keimzellentumor, Knochenkrebs, kolorektales Karzinom, Kopf-Hals-Tumore, Kolonkarzinom, Kraniopharyngeome, Krebs im Mundbereich und auf der Lippe, Leberkrebs, Lebermetastasen, Lidtumor, Lungenkrebs, Lymphdrüsenkrebs, Lymphome, Magenkrebs, malignes Melanom, malignes Neoplasma, Malignome des Magen-Darm-Traktes, Mammakarzinom, Mastdarmkrebs, Medulloblastome, Melanom, Meningeome, Morbus Hodgkin, Mycosis fungoides, Nasenkrebs, Neurinom, Neuroblastom, Nierenkrebs, Nierenzellkarzinome, Non-Hodgkin-Lymphome, Oligodendrogliom, Ösophaguskarzinom, osteolytische Karzinome, osteoplastische Karzinome, Osteosarkom, Ovarial-Karzinom, Pankreaskarzinom, Peniskrebs, Plasmozytom, Plattenepithelkarzinome des Kopfes und Halses, Prostatakrebs, Rachenkrebs, Rektumkarzinom, Retinoblastom, Scheidenkrebs, Schilddrüsenkarzinom, Schneeberger Krankheit, Speiseröhrenkrebs, Spinaliom, T-Zell-Lymphom, Thymom, Tubenkarzinom, Tumoren des Auges, Urethrakrebs, Urologische Tumoren, Urothelkarzinom, Vulvakrebs, Warzenbeteiligung, Weichteiltumoren, Weichteilsarkom, Wilms Tumor, Zervixkarzinom, Zungenkrebs; rheumatoide Arthritis, chronische Lungenentzündung, Asthma, Lupus, Arteriosklerose, Nephritis, Psoriasis, Allergien, Crohn'sche Krankheit, Ischämie, Bowel Krankheit, Tuberkulose, interstitiale Cystitis, Sonnenbrand, Alzheimer, Parkinson Krankheit, Huntington Krankheit, amyotrophische laterale Sclerose, Retinitis pigmentosa, Shy Drager Syndrom, Autoimmunerkrankungen, Osteoporose, ulcerative Colitis, Ekzeme, multiple Sclerosis, Diabetes Mellitus Typ 1, Diabetes Mellitus Typ 2, HIV, AIDS, Varicella-Zoster-Virus-Infektionen, Creutzfeldt-Jakob Krankheit, Encephalitis, Epstein-Barr-Virus-Infektionen, Dermatitis, Helicobacter pylori Infektionen, Herpesvirus-Infektionen, Influenza, Meningitis, Ulcer, Herzinfarkt, Herzinsuffizienz, Angina Pectoris, Arteriosclerose, kongenitale Herzfehler, Hematome, pulmonare Hypertension, Myocarditis, Pericarditis, Phlebitis, Restenose, Stenose, Thrombose, Colon irritabile, Konjunktivitis, Konjunktivitis sicca, Macula degeneration, Tinnitus und Hörschädigung.

9. Kosmetische Zusammensetzung zur nichttherapeutischen Anwendung bei trockener Haut, Stoffwechselstörungen der Haut, Akne, geschädigter Haut, Alterungserscheinungen sowie zur Glättung der Haut und zur Steigerung der Feuchtigkeitsversorgung der Haut enthaltend den entfärbten Extrakt gemäß Anspruch 4.

10. Pharmazeutische Zusammensetzung enthaltend den entfärbten Extrakt gemäß Anspruch 4 zusammen mit mindestens einem pharmakologisch verträglichen Hilfsstoff, Trägerstoff und/oder Lösungsmittel.

11. Pharmazeutische Zusammensetzung gemäß Anspruch 10 für die orale, parenterale und insbesondere für die topische Anwendung.

12. Pharmazeutische Zusammensetzung gemäß Anspruch 10 oder 11 des weiteren enthaltend mindestens ein Vitamin und/oder mindestens einen antiproliferativen, antiangiogenen, antiinflammatorischen, antiphlogistischen, cytostatischen und/oder cytotoxischen Wirkstoff.

13. Pharmazeutische Zusammensetzung gemäß Anspruch 12, wobei der mindestens eine antiinflammatorische Wirkstoff ausgewählt wird aus der Gruppe umfassend oder bestehend aus: Alcofenac, Aceclofenac, Sulindac, Tolmetin, Etodolac, Fenopren, Thiaprofensäure, Meclofenamsäure, Meloxicam, Tenoxicam, Lornoxicam, Nabumeton, Acetaminophen, Phenacetin, Ethenzamid, Sulpyrin, Mefanamsäure, Flufenamsäure, Diclofenacnatrium, Loxoprofennatrium, Phenylbutazon, Indomethacin, Ibuprofen, Ketoprofen, Naproxen, Oxaprozin, Flurbiprofen, Fenbufen, Pranoprofen, Floctafenin, Piroxicam, Epirizol, Tiaramidhydrochlorid, Zaltoprofen, Gabexatmesylat, Camostatmesylat, Ulinastatin, Colchicin, Probenecid, Sulfinpyrazon, Benzbromaron, Allopurinol, Salicyläure, Atropin, Scopolamin, Levorphanol, Ketorolac, Tebufelon, Tenidap, Clofezon, Oxyphenbutazon, Prexazon, Apazon, Benzydamin, Bucolom, Cinchopen, Clonixin, Ditrazol, Epirizol, Fenoprofen, Floctafeninl, Glaphenin, Indoprofen, Nifluminsäure, Suprofen, Bufexamac, Dexamethason, Hexestrol, Methimazol, Betamethason, Triamcinolon, Fluocinonid, Prednisolon, Methylprednisolon, Hydrocortison, Fluorometholon, Beclomethasondipropionat, Estriol, Clobetasol, Diflorasondiacetat, Halbetosalpropionat, Amicinonid, Desoximetason, Halcinonid, Mometasonfuroat, Fluticasonpropionat, Flurandrenolid, Clocortalon, Predincarbat, Aclometasondipropionat oder Desonid.

14. Pharmazeutische Zusammensetzung nach Anspruch 12, wobei der mindestens eine antiproliferative, antiangiogene, antiphlogistische, zytostatische und/oder zytotoxische Wirkstoff aus der Gruppe ausgewählt wird umfassend oder bestehend aus: Chlorethamin, Cyclophosphamid, Trofosfamide, Ifosfamid, Melphalan, Chlorambucil, Busulfan, Thiotepa, Carmustin, Lomustin, Dacarbazin, Procarbazin, Temozolomid, Treosulfan, Estramustin, Nimustin, Daunorubicin, Doxorubicin, Adriamycin, Dactinomycin, Mitomycin C, Bleomycin, Epirubicin, Idarubicin, Dactinomycin, Mitoxantron, Mitomycin-C, Plicamycin, Amsacrin, Actinomycin D, Methotrexat, 5-Fluorouracil, 6-Thioguanin, 6-Mercaptopurin, Fludarabin, Cladribin, Pentostatin, Gemcitabin, Cytarabin, Azathioprin, Raltitrexed, Capecitabin, Cytosinarabinosid, Tioguanin, Mercaptopurin, Vincristin, Vinblastin, Vindesin, Etoposid, Teniposid, Cisplatin, Carboplatin, Oxaliplatin, Vinca-Alkaloide, Venorelbin, Etoposid, Teniposid, Camptothecin, Irinotecan, Paclitaxel, Docetaxel, Hydroxycarbamide, Imatinib, Miltefosin, Amsacrin, Topotecan, Bexaroten, Tretinoin, Asparaginase, Trastuzumab, Alemtuzumab, Rituximab, Glucocorticoide, Prednison Prednisolon, Dexamethason, Oestrogene, Fosfestrol, Estramustin, LHRH, Buserelin, Goserelin, Leuprorelin, Triptorelin, Flutamid, Cyproteronacetat, Tamoxifen, Toremifen, Aminoglutethimid, Formestan, Exemestan, Letrozol, Anastrozol, Interleukin-2, Interferon-$\alpha$, Erythropoietin, G-CSF, Rituximab, Efitinib, Ibritumomab, Levamisol, Indomethacin, Diclofenac, Ketoprofen, Ibuprofen, Flurbiprofen, Nabumeton, Cox-2-Hemmer, Mesalazin, Sulfasalazin, Olsalazin, Infliximab und Retinoide.

**Claims**

1. Process for production of a decolorized extract comprising the following steps:

   I) provision of a colored extract from the fruit of the species Pandanus conoideus,
   II) treatment of the colored extract using:

   a) exposure to UV-light, or
   b) oxidation with peroxides, or
   c) oxidation with oxygen, or
   d) oxidation with reducible halogen compounds, or
   e) hydrogenation with hydrogen and hydrogenation catalysts or

f) biological oxidation with oxidative enzymes.

2. Process according to claim 1, wherein the reducible halogen compound is chlorine water, chlorine, bromine, iodine, HClO (hypochlorous acid), hypochlorite, $ClO_2$, $HClO_2$, chlorite, $HClO_3$, chlorate, $HClO_4$, perchlorate.

3. Process according to claims 1 or 2, wherein the processing of the colored extract is conducted at temperatures lower than 50°C.

4. Decolorized extract obtainable according to the process according to any of claims 1-3.

5. Decolorized extract according to claim 4 for non therapeutic use as appetite suppressant and filling food and for prevention of repeated weight gain after completed diet.

6. Decolorized extract according to claim 4 for use in the treatment of overweight, obesity and excessive food intake.

7. Decolorized extract according to claim 4 for use in the prophylaxis and/or treatment of immune-dermatologic diseases, tumors, cancer, rheumatoid diseases, chronic-recurrent intestine inflammations, degenerative diseases, neurodegenerative diseases, inflammation, gastrointestinal diseases, diabetes, cardiovascular diseases, immunological diseases, infectious diseases, ophthalmologic and otologic diseases.

8. Decolorized extract according to claim 4 for use according to claim 7, wherein the immune-dermatologic diseases, tumors, cancer, rheumatoid diseases, chronic-recurrent intestine inflammations, degenerative diseases, neurodegenerative diseases, inflammation, gastrointestinal diseases, diabetes, cardiovascular diseases, immunological diseases, infectious diseases, ophthalmologic and otologic diseases are selected from the group comprising or consisting of: atopic dermatitis, dermatitis, eczema, psoriasis, psoriatic arthritis, rheumatoid arthritis, osteoarthritis, metabolic disorder of the skin, dry skin, acne, actinic keratosis, acute and chronic myeloid leukemia, adenocarcinoma, choroidal melanoma, acoustic neurinoma, ampullary carcinoma, anal carcinoma, astrocytoma, basalcell carcinoma, pancreatic cancer, sarcoma, bladder cancer, bronchial carcinoma, small-cell and non-small cell lung cancer, breast cancer, Burkitt's lymphoma, endometrial cancer, CUP-syndrome, colorectal cancer, small intestine cancer, small intestinal tumor, ovarian cancer, endometrial carcinoma, ependymoma, epithelial cancer, Ewing's tumors, gastrointestinal tumors, gall bladder carcinoma, gall carcinoma, uterine cancer, cervical cancer, glioblastoma, gynecologic tumors, throat, nose and ear tumors, hematologic neoplasias, hairy cell leukemia, urethral cancer, skin cancer, brain tumor, brain metastasis, testicle cancer, pituitary tumor, carcinoids, Kaposi's sarcoma, laryngeal cancer, germ cell tumor, bone cancer, colorectal carcinoma, head and neck tumors, colon carcinoma, craniopharyngiomas, cancer of mouth area and on lips, liver cancer, liver metastasis, eyelid tumor, lung cancer, lymph node cancer, lymphoma, stomach cancer, malignant melanoma, malignant neoplasia, malignant tumor of gastrointestinal tract, mamma carcinoma, rectal cancer, medulloblastom, melanoma, meningioma, Morbus Hodgkin, mycosis fungoides, nasal cancer, neurinoma, neuroblastoma, kidney cancer, renal cell carcinoma, non-Hodgkin's-lymphoma, oligodendroglioma, esophageal carcinoma, osteolytic carcinoma, osteoplastic carcinoma, osteosarcoma, ovarial carcinoma, pancreatic carcinoma, penile cancer, plasmocytoma, squamous cell carcinoma of the head and neck, prostate cancer, pharyngeal cancer, rectal carcinoma, retinoblastoma, vaginal cancer, thyroid carcinoma, Schneeberger disease, esophageal cancer, spinalioma, T-cell-lymphoma, thymoma, tubal carcinoma, eye tumor, urethral cancer, urologic tumors, urothelial carcinoma, vulva cancer, wart appearance, soft tissue tumor, soft tissue sarcoma, Wilms' tumor, cervical carcinoma, tongue cancer, rheumatoid arthritis, chronic pneumonia, asthma, lupus, arteriosclerosis, nephritis, psoriasis, allergies, Crohn's disease, ischemia, bowel disease, tuberculosis, interstitial cystitis, actinic dermatitis, Alzheimer's disease, Parkinson's disease, Huntington's disease, amyotropic lateral sclerosis, retinitis pigmentosa, Shy-Drager's syndrome, autoimmune diseases, osteoporosis, ulcerative colitis, eczema, multiple sclerosis, diabetes mellitus type 1, diabetes mellitus type 2, HIV, AIDS, varicella-zoster-virus infections, Creutzfeldt Jakob disease, encephalitis, Epstein-Barr-virus infections, dermatitis, *Helicobacter pylori* infections, herpes-virus infections, influenza, meningitis, ulcer, heart attack, cardiac insufficiency, angina pectoris, arteriosclerosis, congenital heart failure, hematoma, pulmonary hypertension, myocarditis, pericarditis, phlebitis, restenosis, stenosis, thrombosis, irritable colon, conjunctivitis, conjunctivitis sicca, macula degeneration, tinnitus and hearing disorders.

9. Cosmetic composition comprising the decolorized extract according to claim 4, for non therapeutic use with dry skin, metabolic disorders of the skin, acne, damaged skin, aging phenomena, as well as for skin smoothing and for increase of skin humidity.

10. Pharmaceutical composition comprising the decolorized extract according to claim 4 together with at least one pharmaceutically compatible adjuvant, carrier and/or solvent.

11. Pharmaceutical composition according to claim 10 for the oral, parenteral and in particular for topical use.

12. Pharmaceutical composition according to claim 10 or 11, which further comprise at least a vitamin and/or at least an antiproliferative, anti-angiogenic, anti-inflammatory, anti-phlogistic, cytostatic and/or cytotoxic agent.

13. Pharmaceutical composition according to claim 12, wherein said at least one anti-inflammatory agent is selected from the group comprising or consisting of:

alcofenac, aceclofenac, sulindac, tolmetin, etolodac, fenopren, thiaprofenic acid, meclofenamic acid, meloxicam, tenoxicam, lornoxicam, nabumeton, acetaminophen, phenacetin, ethenzamide, sulpyrin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenin, piroxicam, epirizol, thiaramide hydrochloride, zaltoprofen, gabexate mesylate, camostate mesylate, ulinastatin, colchicine, probenecid, sulfinpyrazone, benzbromarone, allopurinol, salicylic acid, atropine, scopolamine, levorphanol, ketorolac, tebufelon, tenidap, clofezon, oxyphenbutazone, prexazon, apazon, benzydamine, bucolom, cinchopen, clonixin, ditrazol, epirizol, fenoprofen, floctafenine, glaphenine, indoprofen, niflumic acid, suprofene, bufexemac, dexamethasone, hexestrol, methimazole, betamethasone, triamcinolon, fluocinoncid, prednisolone, methylprednisolone, hydrocortisone, fluorometholon, beclomethasondipropionate, estriol, clobetasol, diflorasondiacetate, halbetosalpropionate, amicinomid, desoximetason, halcinonid, mometasonfuroat, fluticasonpropionate, flurandrenolid, clocortalon, predincarbate, aclometasondipropionate or desonid.

14. Pharmaceutical composition according to claim 13, wherein said at least one antiproliferative, anti-angiogenic, anti-inflammatory, anti-phlogistic, cytostatic and/or cytotoxic agent is selected from the group comprising or consisting of:

chlorethamine, cyclophosphamide, trofosfamide, ifosfamide, melphalan, chlorambucil, busulfan, thiotepa, carmustine, lomustine, dacarbazine, procarbazine, temozolomide, treosulfan, estramustin, nimustin, daunorubicine, doxorubicine, adriamycine, dactinomycine, mitomycine C, bleomycine, epirubicine, idarubicine, dactinomycine, mitoxantron, mytomycine-C, plicamycine, amsacrin, actinomycine D, metotrexate, 5-fluoracil, 6-thioguanine, 6-mercaptopurine, fludarabine, cladribine, pentostatine, gemcitabine, cytabarine, azathioprine, raltitrexed, capecitabine, cytosinarabinoside, tioguanine, mercaptopurine, vincristine, vinblastine, vindesine, etoposide, teniposide, cisplatine, carboplatine, oxaliplatine, vinca-alkaloide, vinorelbine, epotoside, teniposide, camptothecin, irinotecan, paclitaxel, docetaxel, hydroxycarbamide, imatinib, miltefosin, amsacrine, topotecan, bexaroten, tretinoin, asparaginase, trastuzumabe, alemtuzumabe, rituximabe, glucocorticoide, prednisone, prednisolone, dexamethasone, oestrogene, fosfestrole, estramustine, LHRH, buserelin, goserelin, leuprorelin, triptorelin, flutamide, cyproteronacetate, tamoxifen, toremifen, aminoglutethimide, formestan, exemestan, letrozole, anastrozole, interleukine-2, interferon-$\alpha$, erythropoietin, G-CSF, rituximabe, gefitinib, ibritumomab, levamisole, indomethacin, diclofenac, ketoprofen, ibuprofen, flurbiprofen, nabumeton, Cox-2-inhibitors, mesazaline, sulfasalazine, olsalazine, infliximab and retionoide.

**Revendications**

1. Processus pour la production d'un extrait décoloré contenant les étapes suivantes:

1) provision d'un extrait coloré a partir du fruit de l'espèce Pandanus conoideus,
11) traitement de l'extrait coloré par:

a) exposition a la lumière UV, ou
b) oxydation par des peroxydes,
c) oxydation par l'oxygène, ou
d) oxydation avec des composés halogénés réductibles, ou
e) hydrogénation avec de l'hydrogène et des catalyseurs d'hydrogénation ou
f) oxydation biologique par des oxydases.

2. Processus selon la revendication 1, ou ledit composé halogéné réductible est choisi parmi l'eau chlorée, le dichlore, le dibrome, le diiode, HClO (l'acide hypochloreux), l'hypochlorite, $ClO_2$, $HClO_2$, le chlorite, $HClO_3$, le chlorate, $HClO_4$,

le perchlorate.

3. Processus selon les revendications 1 ou 2, ou ledit traitement de l'extrait coloré est conduit à des températures inferieures a 50 °C.

4. Extrait décoloré disponible selon le processus selon chacune des revendications 1-3.

5. Extrait décoloré selon revendication 4 à l'usage non thérapeutique comme suppresseur de l'appétit ou comme nourriture rassasiante et pour la prévention de la prise répétée de poids après avoir effectuée une diète.

6. Extrait décoloré selon revendication 4 pour l'usage dans le traitement du surpoids, de l'obésité et de la consommation alimentaire excessive.

7. Extrait décoloré selon revendication 4 pour l'usage dans la prophylaxie et/ou le traitement des maladies immuno-dermatologiques, des tumeurs, du cancer, des maladies rhumatoïdes, des inflammations chroniques-récurrents des intestins, des maladies dégénératives, des maladies neurodégéneratives, de 1' inflammation, des maladies gastro-intestinales, du diabète, des maladies cardiovasculaires, des maladies immunologiques, des maladies infectieuses, des maladies ophtalmologiques et otologiques.

8. Extrait décoloré selon la revendication 4 a l'usage selon la revendication 7, ou lesdites maladies immuno-dermatologiques, tumeurs, cancer, maladies rhumatoïdes, inflammations chroniques-récurrents des intestines, maladies dégénératives, maladies neurodégénératives, inflammations, maladie gastro-intestinales, diabètes, maladies cardiovasculaires, maladies immunologiques, maladies infectieuses, maladies cardiovasculaires, maladies ophtalmologiques et otologiques sont choisis parmi la dermatite atopique, la dermatite, l'eczéma, le psoriasis, l'arthrite psoriasique, l'arthrite rhumatoïde, l'ostéo-arthrite, les troubles métaboliques de la peau, la peau sèche, l'acné, la kératose actinique, la leucémie myéloïde aiguée et chronique, l'adéno-carcinome, le mélanome choroïdien, le neurinome acoustique, le carcinome papillaire, le carcinome de l'anus, l'astrocytome, le carcinome des cellules basiques, le cancer du pancréas, le sarcome, le cancer de la vessie, le carcinome bronchique, le carcinome des petites cellules et des non à petites cellules du poumon, le cancer du sein, le lymphome de Burkitt, le cancer de l'endomètre, le syndrome du cancer d'origine primaire inconnue, le cancer colorectal, le cancer de l'intestin grêle, la tumeur de l'intestine grêle, le cancer de l'ovaire, le carcinome de l'endomètre, l'épendymome, le cancer épithélial, les tumeurs d'Ewing, les tumeurs gastro-intestinales, le carcinome de la vésicule biliaire, le carcinome de la bille, le cancer utérin, le cancer du cervix, le glioblastome, les tumeurs gynécologiques, les tumeurs de la gorge, du nez et de l'oreille, la néoplasie hématologique, la leucémie des cellules ciliées, le cancer de l'urètre, le cancer de la peau, la tumeur du cerveau, la métastase du cerveau, le cancer du testicule, la tumeur pituitaire, la tumeur carcinoïde, le sarcome de Kaposi, le cancer du larynx, la tumeur de la cellule germinale, le cancer de l'os, le carcinome Colorectal, les tumeurs de la tête et du cou, le carcinome du colon, le craniopharyngiome, le cancer de la région de la bouche et des lèvres, le cancer du foie, la métastase du foie, la tumeur de la paupière, le cancer du poumon, le cancer des ganglions lymphatiques, le lymphome, le cancer de l'estomac, le mélanome malin, la néoplasie maligne, la tumeur maligne du tract gastro-intestinal, le carcinome mammaire, le cancer rectal, le médulloblastome, le mélanome, le méningiome, Morbus Hodgkin, la mycose fongoïde, le cancer nasale, le neurinome, le neuroblastome, le cancer des reins, le cancer des cellules rénales, le lymphome non hodgkinien, l'oligodendrogliome, le carcinome de l'oesophage, le carcinome ostéolytique, le carcinome ostéoplastique, l'ostéosarcome, le carcinome ovarien, le carcinome pancréatique, le cancer du pénis, le plastocytome, le carcinome épidermoïde de la tête et duc coup, le cancer de prostate, le cancer du pharynx, le carcinome rectale, le rétinoblastome, le cancer vaginale, le carcinome thyroïdien, la maladie schneeberger, le cancer oesophagien, le spinaliome, le cancer des cellules T, le thymome, le carcinome tubaire, la tumeur de l'oeil, le cancer urétral, les tumeurs urologiques, le carcinome urothélial, le cancer vulvaire, l'apparition des verres, la tumeur du tissue mou, le sarcome du tissue mou, la tumeur de Wilms, le carcinome cervicale, le cancer de la langue, l'arthrite rhumatoïde, la pneumonie chronique, l'asthme, le lupus, l'artériosclérose, la néphrite, le psoriasis, les allergies, la maladie de Crohn, l'ischémie, la maladie de l'intestin, la tuberculose, la cystite interstitielle, la dermatite actinique, la maladie d'Alzheimer, la maladie de Parkinson, la maladie de Huntington, la sclérose amyotrophique latérale, la rétinite pigmentaire, le syndrome de Shy-Drager, les maladies auto immunes, l'ostéoporose, la rectocolite hémorragique, l'eczéma, la sclérose multiple, le diabète de type 1, le diabète de type 2, le HIV, l'AIDS, les infections par le virus de la varicelle et le virus de l'herpès, la maladie de Creutzfeldt Jacob, l'encéphalite, les infections par le virus Epstein-Barr, la dermatite, les infections à *Helicobacter pylori,* les infections par le virus de l'herpès, la grippe, la méningite, l'ulcère, la crise cardiaque, l'insuffisance cardiaque, l'angine pectorale, l'artériosclérose, l'insuffisance cardiaque congestive, l'hématome, l'hypertension pulmonaire, la myocardite, la péricardite, la phlébite, la resténose, la sténose, la thrombose, le colon irritable, la conjonctivite, la conjonctivite sicca, la dégénération de la macula, l'acouphène, les troubles d'audition.

9. Composition cosmétique comprenant l'extrait décoloré selon la revendication 4, a l'usage non thérapeutique pour la peau sèche, les troubles métaboliques de la peau, l'acné, la peau endommagée, les phénomènes de vieillissement, ainsi que pour le lissage de la peau et pour l'augmentation de l'humidité de la peau.

10. Composition pharmaceutique contenant l'agent décoloré selon la revendication 4 en association avec au moins un pharmacéutiquement acceptable adjuvant, véhicule et/ou solvant.

11. Composition pharmaceutique selon la revendication 10 pour l'usage orale, parentéral et particulièrement topique.

12. Composition pharmaceutique selon la revendication 10 ou 11, qui comprennent de plus au moins une vitamine et/ou au moins un agent antiprolifératif, anti-angiogénique, anti-inflammatoire, anti-phlogistique, cytostatique et/ou cytotoxique.

13. Composition pharmaceutique selon la revendication 12, ou ledit au moins un agent anti-inflammatoire est choisi parmi alcofenac, aceclofenac, sulindac, tolmetin, etolodac, fenopren, l'acide thiaprofénique, l'acide méclofénamique, meloxicam, tenoxicam, lornoxicam, nabumeton, acetaminophene, phénacétine, ethenzamide, sulpyrine, l'acide méfénamique, l'acide flufénamique, diclofenac sodium, loxoprofene sodium, phenylbutazone, indomethacin, ibuprofen, ketoprofen, naproxen, oxaprozine, flurbiprofen, fenbufen, pranoprofen, floctafenine, piroxicam, epirizol, 4-((5-chloro-2-oxo-3(2H)-benzothiazol-yl) acetyl)-1-piperazineethanol hydrochloride, zaltoprofen, gabexate mesylate, camostate mesylate, ulinastatine, colchicine, probenecid, sulfinpyrazone, benzbromarone, allopurinol, l'acide salicylique, atropine, scopolamine, levorphanol, ketorolac, tebufelon, tenidap, clofezon, oxyphenbutazone, prexazon, apazon, benzydamine, bucolom, cinchopen, clonixin, ditrazol, epirizol, fenoprofen, floctafenine, glaphenine, indoprofen, l'acide niflumique, suprofene, bufexemac, dexamethasone, hexestrol, methimazole, betamethasone, triamcinolon, fluocinoncid, prednisolone, methylprednisolone, hydrocortisone, fluorometholon, beclomethasondipropionate, estriol, clobetasol, diflorasondiacetate, halbetosalpropionate, amicinomid, desoximetason, halcinonid, mometasonfuroat, fluticasonpropionate, flurandrenolid, clocortalon, predincarbate, aclometasondipropionate ou desonid.

14. Composition pharmaceutique selon la revendication 13, ou ledit au moins un agent antiproliferative, anti-angiogénique, anti-inflammatoire, anti-phlogistique, cytostatique et/ou cytotoxique est choisi parmi chlorethamine, cyclophosphamide, trofosfamide, ifosfamide, melphalan, chlorambucil, busulfan, thiotepa, carmustine, lomustine, dacarbazine, procarbazine, temozolomide, tréosulfan, estramustine, nimustine, daunorubicine, doxorubicine, adriamycine, dactinomycine, mitomycine C, bleomycine, epirubicine, idarubicine, dactinomycine, mitoxantrone, mytomycine-C, plicamycine, amsacrine, actinomycine D, metotrexate, 5-fluoracil, 6-thioguanine, 6-mercaptopurine, fludarabine, cladribine, pentostatine, gemcitabine, cytabarine, azathioprine, raltitrexed, capecitabine, cytosinarabinoside, tioguanine, mercaptopurine, vincristine, vinblastine, vindesine, etoposide, teniposide, cisplatine, carboplatine, oxaliplatine, vinca-alkaloide, vinorelbine, epotoside, teniposide, camptothécine, irinotecan, paclitaxel, docetaxel, hydroxycarbamide, imatinibe, miltefosine, amsacrine, topotecan, bexaroten, tretinoin, asparaginase, trastuzumabe, alemtuzumabe, rituximabe, glucocorticoide, prednisone, prednisolone, dexamethasone, oestrogene, fosfestrole, estramustine, LHRH, buséréline, goséréline, leuproréline, triptoréline, flutamide, cyproteronacetate, tamoxifen, toremifen, aminoglutethimide, formestane, exemestane, letrozole, anastrozole, l'interleucine-2, l'interféron-$\alpha$, l'érythropoïétine, G-CSF, rituximabe, géfitinib, ibritumomab, lévamisole, indomethacin, diclofenac, ketoprofen, ibuprofen, flurbiprofen, nabumeton, l'inhibiteur de Cox-2, mésazaline, sulfasalazine, olsalazine, infliximab et retionoide.